# EUROPEAN PATENT SPECIFICATION

(11) **EP 0 573 465 B1**
(45) Date of publication and mention of the grant of the patent: **02.04.1997**
(21) Application number: 92904841.1
(22) Date of filing: 15.01.1992
(51) Int. Cl.: A61K 31/20

(54) **THERAPEUTIC COMPOSITIONS TO PROTECT AND RESUSCITATE MAMMALIAN CELLS AND METHODS FOR PREPARING AND USING SAME**
THERAPEUTISCHE ZUSAMMENSETZUNGEN ZUM SCHUTZ UND ZUR WIEDERBELEBUNG VON SÄUGETIERZELLEN UND VERFAHREN ZU DEREN HERSTELLUNG UND ANWENDUNG
COMPOSITIONS THERAPEUTIQUES DE PROTECTION ET DE REGENERATION CELLULAIRE CHEZ LES MAMMIFERES ET PROCEDES DE PREPARATION ET D'UTILISATION

(30) Priority: 01.03.1991 US 663500
(43) Date of publication of application: 15.12.1993
(73) Proprietor: WARNER-LAMBERT COMPANY, Morris Plains New Jersey 07950 (US)
(72) Inventor: MARTIN, Alain, Ringoes, NJ 08851 (US)
(74) Representative: Mansmann, Ivo
(86) International application number: US9200249
(87) International publication number: WO9215292

(56) References cited:
- EP-A- 0 347 056
- DE-A- 3 719 097
- Biological Abstracts, vol. 75, 1983, Biological Abstract Inc., Philadelphia, PA, US; H. WOLTERS et al.: "Radiation effects on membranes: 3. The effect of X irradiation on survival of mammalian cells substituted by polyunsaturated fatty acids", abstract no. 68500

## Description

### BACKGROUND OF THE INVENTION

### 1. Field of the Invention

This invention pertains to therapeutic compositions for protecting and resuscitating mammalian cells. In one embodiment, the therapeutic composition comprises (a) pyruvate, (b) an antioxidant, and (c) a mixture of saturated and unsaturated fatty acids. This invention also pertains to methods for preparing and using the therapeutic compositions and the topical and ingestible pharmaceutical products in which the therapeutic compositions may be used.

### 2. Description of the Prior Art

Mammalian cells are continuously exposed to activated oxygen species such as superoxide (O₂⁻), hydrogen peroxide (H₂O₂), hydroxyl radical (OH^{·}), and singlet oxygen (¹O₂). *In vivo*, these reactive oxygen intermediates are generated by cells in response to aerobic metabolism, catabolism of drugs and other xenobiotics, ultraviolet and x-ray radiation, and the respiratory burst of phagocytic cells (such as white blood cells) to kill invading bacteria. Hydrogen peroxide, for example, is produced during respiration of most living organisms especially by stressed and injured cells.

These active oxygen species can injure cells. An important example of such damage is lipid peroxidation which involves the oxidative degradation of unsaturated lipids. Lipid peroxidation is highly detrimental to membrane structure and function and can cause numerous cytopathological effects. Cells defend against lipid peroxidation by producing radical scavengers such as superoxide dismutase, catalase, and peroxidase. Injured cells have a decreased ability to produce radical scavengers. Excess hydrogen peroxide can react with DNA to cause backbone breakage, produce mutations, and alter and liberate bases. Hydrogen peroxide can also react with pyrimidines to open the 5, 6-double bond, which reaction inhibits the ability of pyrimidines to hydrogen bond to complementary bases, Hallaender et al. (1971). Such oxidative biochemical injury can result in the loss of cellular membrane integrity, reduced enzyme activity, changes in transport kinetics, changes in membrane lipid content, and leakage of potassium ions, amino acids, and other cellular material.

The production of reactive oxygen intermediates has been suggested to cause many skin, tissue, and organ disorders such as atherosclerosis, arthritis, cytotoxicity, skin inflammation, photoaging, wrinkling, actinic keratosis, tumor formation, cancer, hypertension, Parkinson's disease, lung disease, and heart disease. The role of active oxygen radicals in promoting tumors has been proposed based on the findings that (a) tumor promoters increase the level of oxygen radicals, (b) many free radical generating systems promote tumors, and (c) certain antioxidants inhibit the biochemical effects of tumor promoters.

In vitro, reactive oxygen intermediates can be generated in cellular culture media by autooxidation and photooxidation of media components. During excision and storage, transplant organs can suffer oxidative injuries which result in the loss of cellular membrane integrity and shorten the usable life of the organ.

When cells are stressed by oxidative injury, a resuscitation step is necessary to recondition the cells. Antioxidants have been shown to inhibit damage associated with active oxygen species. For example, pyruvate and other *alpha*-ketoacids have been reported to react rapidly and stoichiometrically with hydrogen peroxide to protect cells from cytolytic effects, O'Donnell-Tormey et al., J. Exp. Med., 165, pp. 500-514 (1987).

United States patents nos. 3,920,835, 3,984,556, and 3,988,470, all issued to Van Scott et al., disclose methods for treating acne, dandruff, and palmar keratosis, respectively, which consist of applying to the affected area a topical composition comprising from about 1% to about 20% of a lower aliphatic compound containing from two to six carbon atoms selected from the group consisting of *alpha*-hydroxyacids, *alpha*-ketoacids and esters thereof, and 3-hydroxybutryic acid in a pharmaceutically acceptable carrier. The aliphatic compounds include pyruvic acid and lactic acid.

United States patents nos. 4,105,783 and 4,197,316, both issued to Yu et al., disclose a method and composition, respectively, for treating dry skin which consists of applying to the affected area a topical composition comprising from about 1% to about 20% of a compound selected from the group consisting of amides and ammonium salts of *alpha*-hydroxyacids, *beta*-hydroxyacids, and *alpha*-ketoacids in a pharmaceutically acceptable carrier. The compounds include the amides and ammonium salts of pyruvic acid and lactic acid.

United States patent no. 4,234,599, issued to Van Scott et al., discloses a method for treating actinic and nonactinic skin keratoses which consists of applying to the affected area a topical composition comprising an effective amount of a compound selected from the group consisting of *alpha*-hydroxyacids, *beta*-hydroxyacids, and *alpha*-ketoacids in a pharmaceutically acceptable carrier. The acidic compounds include pyruvic acid and lactic acid.

United States patent no. 4,294,852, issued to Wildnauer et al., discloses a composition for treating skin which comprises the *alpha*-hydroxyacids, *beta-*hydroxyacids, and *alpha*-ketoacids disclosed above by Van Scott et al. in combination with C₃-C₈ aliphatic alcohols.

United States patent no. 4,663,166, issued to Veech, discloses an electrolyte solution which comprises a mixture of L-lactate and pyruvate in a ratio from 20:1 to 1:1, respectively, or a mixture of D-*beta-*hydroxybutyrate and acetoacetate, in a ratio from 6:1 to 0.5:1, respectively.

Sodium pyruvate has been reported to reduce the number of erosions, ulcers, and hemorrhages on the gastric mucosa in guinea pigs and rats caused by acetylsalicylic acid. The analgesic and antipyretic properties of acetylsalicylic acid were not impaired by sodium pyruvate, Puschmann, Arzneimittelforschung, 33, pp. 410-415 and 415-416 (1983).

Pyruvate has been reported to exert a positive inotropic effect in stunned myocardum, which is a prolonged ventricular dysfunction following brief periods of coronary artery occlusions which does not produce irreversible damage, Mentzer et al., Ann. Surg., 209, pp. 629-633 (1989).

Pyruvate has been reported to produce a relative stabilization of left ventricular pressure and work parameter and to reduce the size of infarctions. Pyruvate improves resumption of spontaneous beating of the heart and restoration of normal rates and pressure development, Bunger et al., J. Mol. Cell. Cardiol., 18, pp. 423-438 (1986), Mochizuki et al., J. Physiol. (Paris), 76, pp. 805-812 (1980), Regitz et al., Cardiovasc. Res., 15, pp. 652-658 (1981), Giannelli et al., Ann. Thorac. Surg., 21, pp. 386-396 (1976).

Sodium pyruvate has been reported to act as an antagonist to cyanide intoxification (presumably through the formation of a cyanohydrin) and to protect against the lethal effects of sodium sulfide and to retard the onset and development of functional, morphological, and biochemical measures of acrylamide neuropathy of axons, Schwartz et al., Toxicol. Appl. Pharmacol., 50, pp. 437-442 (1979), Sabri et al., Brain Res., 483, pp. 1-11 (1989).

A chemotherapeutic cure of advanced L1210 leukemia has been reported using sodium pyruvate to restore abnormally deformed red blood cells to normal. The deformed red blood cells prevented adequate drug delivery to tumor cells, Cohen, Cancer Chemother. Pharmacol., 5, pp. 175-179 (1981).

Primary cultures of heterotopic tracheal transplant exposed *in vivo* to 7, 12-dimethylbenz(a)anthracene were reported to be successfully maintained in enrichment medium supplemented with sodium pyruvate along with cultures of interleukin-2 stimulated peripheral blood lymphocytes, and plasmacytomas and hybridomas, pig embryos, and human blastocysts, Shacter, J. Immunol. Methods, 99, pp. 259-270 (1987), Marchok et al., Cancer Res., 37, pp. 1811-1821 (1977), Davis, J. Reprod. Fertil. Suppl., 33, pp. 115-124 (1985), Okamoto et al., No To Shinkei, 38, pp. 593-598 (1986), Cohen et al., J. In Vitro Fert. Embryo Transfer, 2, pp. 59-64 (1985).

United States patents nos. 4,158,057, 4,351,835, 4,415,576, and 4,645,764, all issued to Stanko, disclose methods for preventing the accumulation of fat in the liver of a mammal due to the ingestion of alcohol, for controlling weight in a mammal, for inhibiting body fat while increasing protein concentration in a mammal, and for controlling the deposition of body fat in a living being, respectively. The methods comprise administering to the mammal a therapeutic mixture of pyruvate and dihydroxyacetone, and optionally riboflavin. United States patent no. 4,548,937, issued to Stanko, discloses a method for controlling the weight gain of a mammal which comprises administering to the mammal a therapeutically effective amount of pyruvate, and optionally riboflavin. United States patent no. 4,812,479, issued to Stanko, discloses a method for controlling the weight gain of a mammal which comprises administering to the mammal a therapeutically effective amount of dihydroxyacetone, and optionally riboflavin and pyruvate.

Rats fed a calcium-oxalate lithogenic diet including sodium pyruvate were reported to develop fewer urinary calculi (stones) than control rats not given sodium pyruvate, Ogawa et al., Hinyokika Kiyo, 32, pp. 1341-1347 (1986).

United States patent no. 4,521,375, issued to Houlsby, discloses a method for sterilizing surfaces which come into contact with living tissue. The method comprises sterilizing the surface with aqueous hydrogen peroxide and then neutralizing the surface with pyruvic acid.

United States patent no. 4,416,982, issued to Tauda et al., discloses a method for decomposing hydrogen peroxide by reacting the hydrogen peroxide with a phenol or aniline derivative in the presence of peroxidase.

United States patent no. 4,696,917, issued to Lindstrom et al., discloses an irrigation solution which comprises Eagle's Minimum Essential Medium with Earle's salts, chondroitin sulfate, a buffer solution, 2-mercaptoethanol, and a pyruvate. The irrigation solution may optionally contain ascorbic acid and *alpha-*tocopherol. United States patent no. 4,725,586, issued to Lindstrom et al., discloses an irrigation solution which comprises a balanced salt solution, chondroitin sulfate, a buffer solution, 2-mercaptoethanol, sodium bicarbonate or dextrose, a pyruvate, a sodium phosphate buffer system, and cystine. The irrigation solution may optionally contain ascorbic acid and *gamma*-tocopherol.

United States patent no. 3,887,702 issued to Baldwin, discloses a composition for treating fingernails and toenails which consists essentially of soybean oil or sunflower oil in combination with Vitamin E.

United States patent no. 4,847,069, issued to Bissett et al., discloses a photoprotective composition comprising (a) a sorbohydroxamic acid, (b) an anti-inflammatory agent selected from steroidal anti-inflammatory agents and a natural anti-inflammatory agent, and (c) a topical carrier. Fatty acids may be present as an emollient. United States patent no. 4,847,071, issued to Bissett et al., discloses a photoprotective composition comprising (a) a tocopherol or tocopherol ester radical scavenger, (b) an anti-inflammatory agent selected from steroidal anti-inflammatory agents and a natural anti-inflammatory agent, and (c) a topical carrier. United States patent no. 4,847,072, issued to Bissett et al., discloses a topical composition comprising not more than 25% tocopherol sorbate in a topical carrier.

United States patent no. 4,533,637, issued to Yamane et al., discloses a culture medium which comprises a carbon source, a nucleic acid source precursor, amino acids, vitamins, minerals, a lipophilic nutrient, and serum albumin, and cyclodextrins. The lipophilic substances include unsaturated fatty acids and lipophilic vitamins such as Vitamin A, D, and E. Ascorbic acid may also be present.

United Kingdom patent application no. 2,196,348A, to Kovar et al., discloses a synthetic culture medium which comprises inorganic salts, monosaccharides, amino acids, vitamins, buffering agents, and optionally sodium pyruvate adding magnesium hydroxide or magnesium oxide to the emulsion. The oil phase may include chicken fat.

United States patent no. 4,284,630, issued to Yu et al., discloses a method for stabilizing a water-in-oil emulsion which comprises adding magnesium hydroxide or magnesium oxide to the emulsion. The oil phase may include chicken fat.

Preparation-H has been reported to increase the rate of wound healing in artificially created rectal ulcers. The active ingredients in Preparation-H are skin respiratory factor and shark oil, Subramanyam et al., Digestive Diseases and Sciences, 29, pp. 829-832 (1984).

The addition of sodium pyruvate to bacterial and yeast systems has been reported to inhibit hydrogen peroxide production, enhance growth, and protect the systems against the toxicity of reactive oxygen intermediates. The optimum ratio of unsaturated to saturated fatty acids contained within chicken fat enhanced membrane repair and reduced cytotoxicity. The antioxidants glutathione and thioglycollate reduced the injury induced by oxygen radical species, Martin, Ph.D. thesis, (1987-89).

While the above therapeutic compositions are reported to inhibit the production of reactive oxygen intermediates, none of the above compositions are entirely satisfactory. None of the compositions has the ability to simultaneously decrease cellular levels of hydrogen peroxide production, increase cellular resistance to cytotoxic agents, increase rates of cellular proliferation, and increase cellular viability to protect and resuscitate mammalian cells. The present invention provides such improved therapeutic compositions without the disadvantages characteristic of previously known compositions. This invention also relates to methods for preparing and using the therapeutic compositions and the topical and ingestible pharmaceutical products in which the therapeutic compositions may be used.

### SUMMARY OF THE INVENTION

The present invention provides compositions and processes as defined in claims 1 to 26

The present invention pertains to therapeutic compositions for preventing and reducing injury to mammalian cells and increasing the resuscitation rate of injured mammalian cells. The therapeutic compositions comprise (a) pyruvate selected from the group consisting of pyruvic acid, pharmaceutically acceptable salts of pyruvic acid, and mixtures thereof, (b) an antioxidant, and (c) a mixture of saturated and unsaturated fatty acids wherein the fatty acids are those fatty acids required for the resuscitation of injured mammalian cells.

The therapeutic compositions may be utilized in a wide variety of topical and ingestible pharmaceutical products. This invention also relates to methods for preparing and using the therapeutic compositions and the topical and ingestible pharmaceutical products in which the therapeutic compositions may be used.

### BRIEF DESCRIPTION OF THE DRAWINGS

FIGURE 1 depicts in bar graph format the viability of U937 monocytic cells following exposure of the cells to various antioxidants (Examples 1-5).

FIGURE 2 depicts in bar graph format the viability of U937 monocytic cells following exposure of the cells to various combinations of antioxidants (Examples 6-13).

FIGURE 3 depicts in bar graph format the levels of hydrogen peroxide produced by U937 monocytic cells following exposure of the cells to various antioxidants (Examples 14-18).

FIGURE 4 depicts in bar graph format the levels of hydrogen peroxide produced by U937 monocytic cells following exposure of the cells to various combinations of antioxidants (Examples 19-26).

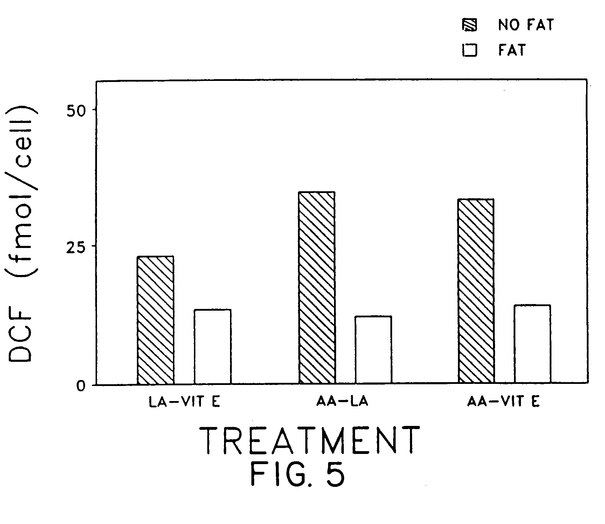

FIGURE 5 depicts in bar graph format the levels of hydrogen peroxide produced by U937 monocytic cells following exposure of the cells to various combinations of antioxidants with and without a mixture of saturated and unsaturated fatty acids (Examples 27-32).

FIGURE 6 depicts in bar graph format the levels of hydrogen peroxide produced by epidermal keratinocytes following exposure of the cells to various antioxidants with and without a mixture of saturated and unsaturated fatty acids (Examples 33-42).

FIGURE 7 depicts in bar graph format the levels of hydrogen peroxide produced by epidermal keratinocytes following exposure of the cells to various combinations of antioxidants with and without a mixture of saturated and unsaturated fatty acids (Examples 43-52).

### DETAILED DESCRIPTION OF THE INVENTION

Applicant has discovered therapeutic compositions for preventing and reducing injury to mammalian cells and increasing the resuscitation rate of injured mammalian cells. Cells treated with the therapeutic compositions of the present invention show decreased levels of hydrogen peroxide production, increased resistance to cytotoxic agents, increased rates of proliferation, and increased viability. Cellular cultures containing the therapeutic compositions showed enhanced differentiation and proliferation over control cultures and rapidly formed attachments or tight junctions between the cells to form an epidermal sheet.

The term "injured cell" as used herein means a cell which has (a) injured membranes so that transport through the membranes is diminished resulting in an increase in toxins and normal cellular wastes inside the cell and a decrease in nutrients and other components necessary for cellular repair inside the cell, (b) an increase in concentration of oxygen radicals inside the cell because of the decreased ability of the cell to produce antioxidants and enzymes, and (c) damaged DNA, RNA, and ribosomes which must be repaired or replaced before normal cellular functions can be resumed. The term "resuscitation" of injured mammalian cells as used herein means the reversal of cytotoxicity, the stabilization of the cellular membrane, an increase in the proliferation rate of the cell, and/or the normalization of cellular functions such as the secretion of growth factors, hormones, and the like. The term "cytotoxicity" as used herein means a condition caused by a cytotoxic agent that injures the cell. Injured cells do not proliferate because injured cells expend all energy on cellular repair. Aiding cellular repair promotes cellular proliferation.

Epidermal keratinocytic cells and monocytic cells have multiple oxygen generating mechanisms and the degree to which each type of mechanism functions differs in each type of cell. In monocytes, for example, the respiratory bursting process is more pronounced than in epidermal keratinocytes. Hence, the components in the therapeutic compositions of the present invention may vary depending upon the types of cells involved in the condition being treated.

The therapeutic composition for treating mammalian cells, preferably epidermal keratinocytes, comprises (a) pyruvate, (b) an antioxidant, and (c) a mixture of saturated and unsaturated fatty acids.

While not wishing to be bound by theory, applicant believes that pyruvate can be transported inside a cell where it can act as an antioxidant to neutralize oxygen radicals in the cell. Pyruvate can also be used inside the cell in the citric acid cycle to provide energy to increase cellular viability, and as a precursor in the synthesis of important biomolecules to promote cellular proliferation. In addition, pyruvate can be used in the multifunction oxidase system to reverse cytotoxicity. Antioxidants, especially lipid-soluble antioxidants, can be absorbed into the cell membrane to neutralize oxygen radicals and thereby protect the membrane. The combination of pyruvate inside the cell and an antioxidant in the cellular membrane functions in a synergistic manner to reduce hydrogen peroxide production in the cell to levels lower than can be achieved by use of either type of component alone.

The saturated and unsaturated fatty acids in the present invention are those fatty acids required for the resuscitation of mammalian cells. Hence, the fatty acids in the therapeutic composition are readily available for the repair of injured cells. Cells injured by oxygen radicals need to produce unsaturated fatty acids to repair cellular membranes. However, the production of unsaturated fatty acids by cells requires oxygen. Thus, the injured cell needs high levels of oxygen to produce unsaturated fatty acids and at the same time needs to reduce the level of oxygen within the cell to reduce oxidative injury. By providing the cell with the unsaturated fatty acids needed for repair, the need of the cell for unsaturated fatty acids is reduced and the need for high oxygen levels is also reduced. The presence of mixtures of saturated and unsaturated fatty acids in the therapeutic composition significantly enhances the ability of pyruvate and the antioxidant to inhibit reactive oxygen production. By stabilizing the cellular membrane, unsaturated fatty acids also improve membrane function and enhance pyruvate transport into the cell. Hence, the three components in the therapeutic composition function together in a synergistic manner to prevent and reduce injury to mammalian cells and increase the resuscitation rate of injured mammalian cells.

Accordingly, the combination of ingredients set out above functions together in an enhanced manner to prevent and reduce injury to mammalian cells and increase the resuscitation rate of injured mammalian cells. The therapeutic effect of the combination of the components in each of the above embodiments is markedly greater than that expected by the mere addition of the individual therapeutic components. Hence, applicant's therapeutic compositions for treating mammalian cells have the ability to decrease intracellular levels of hydrogen peroxide production, increase cellular resistance to cytotoxic agents, increase rates of cellular proliferation, and increase cellular viability.

The cells which may be treated with the therapeutic compositions in the present invention are mammalian cells. Although applicant will describe the present therapeutic compositions as useful for treating mammalian epidermal keratinocytes and mammalian monocytes, applicant contemplates that all mammalian cells which may be protected or resuscitated by applicant's therapeutic compositions may be used in the present invention. Keratinocytes are representative of normal mammalian cells and are the fastest proliferating cells in the body. The correlation between the reaction of keratinocytes to injury and therapy and that of mammalian cells in general is very high. Monocytes are representative of specialized mammalian cells such as the white blood cells in the immune system and the organ cells in liver, kidney, heart, and brain. The mammalian cells may be treated *in vivo* and *in vitro*.

Epidermal keratinocytes are the specialized epithelial cells of the epidermis which synthesize keratin, a scleroprotein which is the principal constituent of epidermis, hair, nails, horny tissue, and the organic matrix of the enamel of teeth. Mammalian epidermal keratinocytes constitute about 95% of the epidermal cells and together with melanocytes form the binary system of the epidermis. In its various successive stages, epidermal keratinocytes are also known as basal cells, prickle cells, and granular cells.

Monocytes are mononuclear phagocytic leukocytes which undergo respiratory bursting and are involved in reactive oxygen mediated damage within the epidermis. Leukocytes are white blood cells or corpuscles which may be classified into two main groups: granular leukocytes (granulocytes) which are leukocytes with abundant granules in the cytoplasm and nongranular leukocytes (nongranulocytes) which are leukocytes without specific granules in the cytoplasm and which include the lymphocytes and monocytes. Phagocyte cells are cells which ingest microorganisms or other cells and foreign particles. Monocytes are also known as large mononuclear leukocytes, and hyaline or transitional leukocytes.

Pyruvic acid (2-oxopropanoic acid, *alpha-*ketopropionic acid, CH₃COCOOH) or pyruvate is a fundamental intermediate in protein and carbohydrate metabolism and in the citric acid cycle. The citric acid cycle (tricarboxylic acid cycle, Krebs cycle) is the major reaction sequence which executes the reduction of oxygen to generate adenosine triphosphate (ATP) by oxidizing organic compounds in respiring tissues to provide electrons to the transport system. Acetyl coenzyme A ("active acetyl") is oxidized in this process and is thereafter utilized in a variety of biological processes and is a precursor in the biosynthesis of many fatty acids and sterols. The two major sources of acetyl coenzyme A are derived from the metabolism of glucose and fatty acids. Glycolysis consists of a series of transformations wherein each glucose molecule is transformed in the cellular cytoplasm into two molecules of pyruvic acid. Pyruvic acid may then enter the mitochondria where it is oxidized by coenzyme A in the presence of enzymes and cofactors to acetyl coenzyme A. Acetyl coenzyme A can then enter the citric acid cycle.

In muscle, pyruvic acid (derived from glycogen) is reduced to lactic acid during exertion. Lactic acid is reoxidized and partially retransformed to glycogen during rest. Pyruvate can also act as an antioxidant to neutralize oxygen radicals in the cell and can be used in the multifunction oxidase system to reverse cytotoxicity.

The pyruvate in the present invention may be selected from the group consisting of pyruvic acid, pharmaceutically acceptable salts of pyruvic acid, and mixtures thereof. In general, the pharmaceutically acceptable salts of pyruvic acid may be alkali salts and alkaline earth salts. Preferably, the pyruvate is selected from the group consisting of pyruvic acid, lithium pyruvate, sodium pyruvate, potassium pyruvate, magnesium pyruvate, calcium pyruvate, zinc pyruvate, manganese pyruvate, and mixtures thereof. More preferably, the pyruvate is selected from the group of salts consisting of sodium pyruvate, potassium pyruvate, magnesium pyruvate, calcium pyruvate, zinc pyruvate, manganese pyruvate, and mixtures thereof. Most preferably, the pyruvate is sodium pyruvate.

The amount of pyruvate present in the therapeutic compositions of the present invention is a therapeutically effective amount. A therapeutically effective amount of pyruvate is that amount of pyruvate necessary to prevent and reduce injury to mammalian cells and increase the resuscitation rate of injured mammalian cells. The exact amount of pyruvate is a matter of preference subject to such factors as the type of condition being treated as well as the other ingredients in the composition. In a preferred embodiment, pyruvate is present in the therapeutic composition in an amount from about 10% to about 50%, preferably from about 20% to about 45%, and more preferably from about 25% to about 40%, by weight of the therapeutic composition.

Antioxidants are substances which inhibit oxidation or suppress reactions promoted by oxygen or peroxides. Antioxidants, especially lipid-soluble antioxidants, can be absorbed into the cellular membrane to neutralize oxygen radicals and thereby protect the membrane. The antioxidants useful in the present invention may be selected from the group consisting of Vitamin A (retinol), Vitamin A₂ (3, 4-didehydroretinol), all forms of carotene such as *alpha*-carotene, *beta-*carotene (*beta*, *beta*-carotene), *gamma*-carotene, *delta*-carotene, Vitamin C (ascorbic acid, L-ascorbic acid), all forms of tocopherol such as Vitamin E (*alpha*-tocopherol, 3,4-dihydro-2,5,7,8-tetramethyl-2-(4,8,12-trimethyltridecyl)-2H-1-benzopyran-6-ol), *beta*-tocopherol, *gamma-*tocopherol, and *delta*-tocopherol, and mixtures thereof. Preferably, the antioxidant is selected from the group of lipid-soluble antioxidants consisting of Vitamin A, *beta-*carotene, Vitamin E, and mixtures thereof. More preferably, the antioxidant is Vitamin E.

The amount of antioxidant present in the therapeutic compositions of the present invention is a therapeutically effective amount. A therapeutically effective amount of antioxidant is that amount of antioxidant necessary to prevent and reduce injury to mammalian cells and increase the resuscitation rate of injured mammalian cells. The exact amount of antioxidant is a matter of preference subject to such factors as the type of condition being treated as well as the other ingredients in the composition. In a preferred embodiment, the antioxidant is present in the therapeutic composition in an amount from about 10% to about 50%, preferably from about 20% to about 45%, and more preferably from about 25% to about 40%, by weight of the therapeutic composition.

The mixture of saturated and unsaturated fatty acids in the present invention are those fatty acids required for the repair of mammalian cellular membranes. Hence, the fatty acids are readily incorporated into the cell and are immediately available for the repair of injured cells. By providing the cell with the unsaturated fatty acids needed for repair, the need of the cell for unsaturated fatty acids is reduced and the need for high oxygen levels is also reduced. Accordingly, the presence of the mixtures of saturated and unsaturated fatty acids in the therapeutic compositions significantly enhances the ability of pyruvate, lactate, and the antioxidant to inhibit reactive oxygen production.

Fatty acids are carboxylic acid compounds found in animal and vegetable fat and oil. Fatty acids are classified as lipids and are composed of chains of alkyl groups containing from 4 to 22 carbon atoms and 0-3 double bonds and characterized by a terminal carboxyl group, -COOH. Fatty acids may be saturated or unsaturated and may be solid, semisolid, or liquid. The most common saturated fatty acids are butyric acid (C₄), lauric acid (C₁₂), palmitic acid (C₁₆), and stearic acid (C₁₈). Unsaturated fatty acids are usually derived from vegetables and consist of alkyl chains containing from 16 to 22 carbon atoms and 0-3 double bonds with the characteristic terminal carboxyl group. The most common unsaturated fatty acids are oleic acid, linoleic acid, and linolenic acid (all C₁₈ acids).

In general, the mixture of saturated and unsaturated fatty acids required for the repair of mammalian cellular membranes in the present invention may be derived from animal fats and waxes. Cells produce the chemical components and the energy required for cellular viability and store excess energy in the form of fat. Fat is adipose tissue stored between organs of the body to furnish a reserve supply of energy. The preferred animal fats and waxes have a fatty acid composition similar to that of human fat and the fat contained in human breast milk. The preferred animal fats and waxes may be selected from the group consisting of human fat, chicken fat, cow fat (defined herein as a bovine domestic animal regardless of sex or age), sheep fat, horse fat, pig fat, and whale fat. The more preferred animal fats and waxes may be selected from the group consisting of human fat and chicken fat. The most preferred animal fat is human fat. Mixtures of other fats and waxes, such as vegetable waxes and synthetic waxes, which have a fatty acid composition similar to that of animal fats and waxes, and preferably to that of human fats and waxes, may also be employed. The mixture of saturated and unsaturated fatty acids may also be derived from shark liver oil.

In a preferred embodiment, the mixture of saturated and unsaturated fatty acids has a composition similar to that of human fat and comprises the following fatty acids: butyric acid, caproic acid, caprylic acid, capric acid, lauric acid, myristic acid, myristoleic acid, palmitic acid, palmitoleic acid, stearic, oleic acid, linoleic acid, linolenic acid, arachidic acid, and gaddoleic acid. Preferably, butyric acid, caproic acid, caprylic acid, capric acid, lauric acid, myristic acid, myristoleic acid, palmitic acid, palmitoleic acid, stearic, oleic acid, linoleic acid, linolenic acid, arachidic acid, and gaddoleic acid are present in the mixture in about the following percentages by weight, respectively (carbon chain number and number of unsaturations are shown parenthetically, respectively): 0.2%-0.4% (C₄), 0.1% (C₆), 0.3%-0.8% (C₈), 2.2%-3.5% (C₁₀), 0.9%-5.5% (C₁₂), 2.8%-8.5% (C₁₄), 0.1%-0.6% (C_{14:1}), 23.2%-24.6% (C₁₆), 1.8%-3.0% (C_{16:1}), 6.9%-9.9% (C₁₈), 36.0%-36.5% (C_{18:1}), 20%-20.6% (C_{18:2}), 7.5-7.8% (C_{18:3}), 1.1%-4.9% (C₂₀), and 3.3%-6.4% (C_{20:1/2}).

In another preferred embodiment, the mixture of saturated and unsaturated fatty acids is chicken fat comprising the following fatty acids: lauric acid, myristic acid, myristoleic acid, pentadecanoic acid, palmitic acid, palmitoleic acid, margaric acid, margaroleic acid, stearic, oleic acid, linoleic acid, linolenic acid, arachidic acid, and gaddoleic acid. Preferably, lauric acid, myristic acid, myristoleic acid, pentadecanoic acid, palmitic acid, palmitoleic acid, margaric acid, margaroleic acid, stearic, oleic acid, linoleic acid, linolenic acid, arachidic acid, and gaddoleic acid are present in the mixture in about the following percentages by weight, respectively: 0.1% (C₁₂), 0.8% (C₁₄), 0.2% (C_{14:1}), 0.1% (C₁₅), 25.3% (C₁₆), 7.2% (C_{16:1}), 0.1% (C₁₇), 0.1% (C_{17:1}), 6.5% (C₁₈), 37.7% (C_{18:1}), 20.6% (C_{18:2}), 0.8% (C_{18:3}), 0.2% (C₂₀), and 0.3% (C_{20:1}).

The above fatty acids and percentages thereof present in the fatty acid mixture are given as an example. The exact type of fatty acid present in the fatty acid mixture and the exact amount of fatty acid employed in the fatty acid mixture may be varied in order to obtain the result desired in the final product and such variations are now within the capabilities of those skilled in the art without the need for undue experimentation.

The amount of fatty acids present in the therapeutic compositions of the present invention is a therapeutically effective amount. A therapeutically effective amount of fatty acids is that amount of fatty acids necessary to prevent and reduce injury to mammalian cells and increase the resuscitation rate of injured mammalian cells. The exact amount of fatty acids employed is subject to such factors as the type and distribution of fatty acids employed in the mixture, the type of condition being treated, and the other ingredients in the composition. In a preferred embodiment, the fatty acids are present in the therapeutic composition in an amount from about 10% to about 50%, preferably from about 20% to about 45%, and more preferably from about 25% to about 40%, by weight of the therapeutic composition.

The present invention extends to methods for making the therapeutic compositions. In general, a therapeutic composition is made by forming an admixture of the components of the composition. The therapeutic composition is made by forming an admixture of (a) a pyruvate, (b) an antioxidant, and (c) a mixture of saturated and unsaturated fatty acids.

For some applications, the admixture may be formed in a solvent such as water. If necessary, the pH of the solvent is adjusted to a range from about 3.5 to about 8.0, and preferably from about 4.5 to about 7.5, and more preferably about 6.0 to about 7.4. The admixture is then sterile filtered. Other ingredients may also be incorporated into the therapeutic composition as dictated by the nature of the desired composition as well known by those having ordinary skill in the art. The ultimate therapeutic compositions are readily prepared using methods generally known in the pharmaceutical arts.

The present invention extends to methods for employing the therapeutic compositions in vitro. In general, a therapeutic composition is employed by contacting the therapeutic composition with mammalian cells.

The therapeutic compositions of the present invention may be utilized in a wide variety of topical and ingestible pharmaceutical products. The therapeutic compositions may be used alone, may be used in combination with medicaments useful for treating injured mammalian cells to prepare augmented pharmaceutical compositions, and may be used in combination with medicaments which are cytotoxic to mammalian cells to prepare cytoprotective pharmaceutical compositions.

The therapeutic composition for treating mammalian cells, preferably epidermal keratinocytes, in combination a medicament comprises:
(a) pyruvate selected from the group consisting of pyruvic acid, pharmaceutically acceptable salts of pyruvic acid, and mixtures thereof;
(b) an antioxidant; and
(c) a mixture of saturated and unsaturated fatty acids wherein the fatty acids are those fatty acids required for membrane repair and stabilization.

In one embodiment, the therapeutic compositions may be utilized by themselves to protect, that is, to prevent and reduce injury to mammalian cells, and increase the resuscitation rate of injured mammalian cells. In a second embodiment, the therapeutic compositions may be combined with a medicament which is useful for treating injured mammalian cells to form augmented pharmaceutical compositions having an enhanced ability to prevent and reduce injury to mammalian cells and further increase the resuscitation rate of injured mammalian cells. In a third embodiment, the therapeutic compositions may be combined with a medicament which is cytotoxic to cells to form cytoprotective pharmaceutical compositions to prevent and reduce injury to mammalian cells from the cytotoxic medicament, increase the resuscitation rate of injured mammalian cells, and thereby increase the therapeutic effect of the cytotoxic medicament. In a further embodiment, therapeutic compositions comprising pyruvate may be combined with a medicament which is cytotoxic to cells to form preventative cytoprotective pharmaceutical compositions for preventing and reducing injury to mammalian cells from the cytotoxic medicament and thereby increasing the therapeutic effect of the cytotoxic medicament. In a further embodiment, the components of the therapeutic compositions may be utilized in wound healing pharmaceutical compositions to increase the resuscitation rate of injured mammalian cells.

The therapeutic compositions may be utilized by themselves in topical products, ingestible products, and tissue culture medium to protect mammalian cells and increase the resuscitation rate of injured mammalian cells. For example, the therapeutic compositions may be used in topical skin care products to protect and increase the resuscitation rate of skin tissue such as in the treatment of various dermatological disorders such as hyperkeratosis, photo-aging, and sunburn photoreactive processes. The topical therapeutic compositions may also be used orally in the form of a mouth wash or spray to protect and accelerate the healing of injured oral tissue such as mouth sores and burns. The topical therapeutic compositions may further be used in ophthalmological preparations such as eye care products to neutralize hydrogen peroxide used in the cleaning of contact lenses. The topical therapeutic compositions may in addition be used in anorectal creams and suppositories to treat such conditions as pruritus ani, proctitis, anal fissures, and hemorrhoids.

The therapeutic compositions may also be used in ingestible products to protect and increase the resuscitation rate of erosions, stomach ulcers, and hamorrhages in the gastric mucosa. Other ingestible therapeutic products include: heart medication to improve regional ventricular function and restore normal heart rate and pressure functions; lung medication to suppress generalized polymorphonuclear cell activation; liver medication to suppress lipogenesis of alcoholic origin and prevent hepatic steatosis; kidney medication to suppress urinary calculi (kidney stones); detoxification medication to antagonize heavy metal poisoning, cyanide poisoning, sodium sulfide poisoning, other types of poisoning; and reduce and neutralize the production of oxygen radicals which produces injury to tissue.

The therapeutic compositions of the present invention may also be used in tissue culture media and organ transplant media to prevent and reduce injury to mammalian cells and increase the resuscitation rate of injured mammalian cells. Tissue cultures and transplant organs encounter reactive oxygen species generated in the culture media by the injured cells. Organs particularly susceptible to oxidative damage during transport and transplantation due to reperfusion injury following ischemia are corneas, livers, hearts, and kidneys. The therapeutic compositions may be useful to abrogate reperfusion injury to such transplant organs.

In the second embodiment, the therapeutic compositions may be combined with a medicament which is useful for treating injured mammalian cells to form topical and ingestible augmented pharmaceutical compositions. In this embodiment, the combination of the therapeutic compositions of the present invention and the medicament useful for treating injured mammalian cells provides an augmented pharmaceutical composition having an enhanced ability to prevent and reduce injury to mammalian cells and further increase the resuscitation rate of injured mammalian cells. The tissue damage associated with many diseases and conditions such as autoimmune disease and benign and malignant skin growths is believed to be caused by the production of cellular produced active oxygen species. Combination of the therapeutic compositions of the present invention and medicaments useful for treating such diseases and conditions may suppress such reactive oxygen-linked tissue injury. For example, the therapeutic compositions may be used in topical augmented pharmaceutical compositions in combination with medicaments such as anti-inflammatories, antibacterials, antiseptics, burn relief medications, sun burn medications, acne preparations, insect bite and sting medications, wound cleansers and wound dressings, to protect and further enhance the resuscitation rate of the injured mammalian cells. The therapeutic compositions may also be used in ingestible augmented pharmaceutical compositions in combination with medicaments used to treat injured mammalian cells such as stroke medications, autoimmune disease medications, arthritis medications, ulcer medications, and cancer medications, to protect and further enhance the resuscitation rate of the injured mammalian cells.

In a specific embodiment, the invention is directed at an augmented pharmaceutical composition having an enhanced ability to prevent and reduce injury to mammalian cells which comprises:
(A) a therapeutic composition selected from the group of consisting of:
   (1)
      (a) pyruvate selected from the group consisting of pyruvic acid, pharmaceutically acceptable salts of pyruvic acid, and mixtures thereof;
      (b) an antioxidant; and
      (c) a mixture of saturated and unsaturated fatty acids wherein the fatty acids are those fatty acids required for the resuscitation of injured mammalian cells;
      and
(B) a medicament useful for treating injured mammalian cells.

In a preferred embodiment, the augmented pharmaceutical composition comprises the therapeutic compositions of the present invention and a medicament useful for treating injured mammalian cells selected from the group consisting of anti-inflammatories and wound cleansers and wound dressings. In a more preferred embodiment, the augmented pharmaceutical composition comprises a medicament selected from the group consisting of wound cleansers and wound dressings.

In the third embodiment, the therapeutic compositions may be combined with a medicament which is cytotoxic to cells to form topical and ingestible cytoprotective pharmaceutical compositions. In this embodiment, the combination of the therapeutic compositions of the present invention and the medicament cytotoxic to cells provides a cytoprotective pharmaceutical composition having an ability to prevent and reduce injury to mammalian cells from a cytotoxic medicament, increase the resuscitation rate of injured mammalian cells, and thereby increase the dose of the cytotoxic medicament. Medicaments taken on a long term regimen tend to cause liver, kidney, tissue, and other toxicity problems. In addition, certain cytotoxic medicaments, such as potent chemotherapeutic medicaments used to treat malignant tissues, are believed to stimulate release of significant amounts of reactive oxygen species by mammalian tissues which can cause oxidative injury. Combination of the therapeutic compositions of the present invention with such cytotoxic medicaments may inhibit induction of reactive oxygen production while simultaneously decreasing side effects of such medicaments. By decreasing the side effects of such medicaments, the dosage levels of the medicaments may be increased thereby increasing the therapeutic effect of the medicaments. For example, the therapeutic compositions may be used in topical cytoprotective pharmaceutical compositions in combination with cytotoxic medicaments such as epithelial cell cohesiveness reducers such as tretinoin (Retin A), dermatological abradants, and anti-inflammatories, to protect and enhance the resuscitation rate of the injured mammalian cells. The therapeutic compositions may also be used in ingestible cytoprotective pharmaceutical compositions in combination with medicaments that cause cytotoxic side effects such as anti-tumor, anti-viral, and antibacterial medicaments including the lipid regulating agents gemfibrozil and lovastatin, centrally acting anticholinesterases such as tacrine, chemotherapeutic medicaments such as the anthracycline antibiotic doxorubicin, gastric irritants such as acetylsalicylic acid and ibuprofen, to protect and enhance the resuscitation rate of the injured mammalian cells.

In a specific embodiment, the invention is directed at a cytoprotective pharmaceutical composition for preventing and reducing injury to mammalian cells from a medicament having cytotoxic properties which comprises:
(A) a therapeutic composition selected from the group consisting of:
   (1)
      (a) pyruvate selected from the group consisting of pyruvic acid, pharmaceutically acceptable salts of pyruvic acid, and mixtures thereof;
      (b) an antioxidant; and
      (c) a mixture of saturated and unsaturated fatty acids wherein the fatty acids are those fatty acids required for the resuscitation of injured mammalian cells;
      and
(B) a medicament having cytotoxic properties.

In a preferred embodiment, the medicament having cytotoxic properties in the cytoprotective pharmaceutical compositions is selected from the group consisting of gemfibrozil, lovastatin, tacrine, and doxorubicin. In a more preferred embodiment, the medicament having cytotoxic properties in the cytoprotective pharmaceutical compositions is selected from the group consisting of gemfibrozil and tacrine. In a most preferred embodiment, the medicament having cytotoxic properties is gemfibrozil. Gemfibrozil (LOPID) is a lipid regulating agent which lowers elevated serum lipids primarily by decreasing serum triglyceride with a variable reduction in total serum cholesterol. Lovastatin (MEVACOR) is a cholesterol lowering agent which inhibits the enzymatic biosynthesis of cholesterol. Tacrine (1,2,3,4-tetrahydro-9-acridinamine) is a centrally active anticholinesterase useful as a cognition activator. Tacrine has undergone clinical trials for use in treating severe Alzheimer's disease (presenile dementia). Doxorubicin (adriamycin) is a cytotoxic anthracycline antibiotic reported to produce regression in disseminated neoplastic conditions such as in various leukemias, tumors, neuroblastomas, sarcomas, and carcinomas.

In another embodiment, therapeutic compositions comprising pyruvate may be combined with a medicament, which is cytotoxic to cells, to form a preventative cytoprotective pharmaceutical composition. These preventative compositions may be used to protect mammalian cells, which have not been injured by the medicament having cytotoxic properties and which do not require resuscitation, and to thereby increase the therapeutic effect of the cytotoxic medicament.

In a specific embodiment, the invention is directed at a preventative cytoprotective pharmaceutical composition for preventing and reducing injury to mammalian cells from a medicament having cytotoxic properties which comprises:
(A) a therapeutic composition comprising pyruvate selected from the group consisting of pyruvic acid, pharmaceutically acceptable salts of pyruvic acid, and mixtures thereof; and
(B) a medicament having cytotoxic properties.

In another form of the fifth embodiment, therapeutic compositions comprising pyruvate may be combined in an immediate release form with an anticancer medicament having cytotoxic properties in a timed release form to provide a timed release preventative cytoprotective pharmaceutical composition. In this embodiment, the timed release composition releases the therapeutic composition substantially immediately and releases the cytotoxic chemotherapeutic medicament after a suitable period of time, for example 15 minutes after releasing the therapeutic composition, to selectively protect non-cancerous cells in the presence of cancerous cells against the cytotoxic chemotherapeutic medicament.

In a specific embodiment, the invention is direct at a timed release preventative cytoprotective pharmaceutical composition for selectively protecting non-cancerous mammalian cells in the presence of cancerous mammalian cells from an anticancer medicament having cytotoxic properties which comprises:
(A) a therapeutic composition in an immediate release form comprising pyruvate selected from the group consisting of pyruvic acid, pharmaceutically acceptable salts of pyruvic acid, and mixtures thereof; and
(B) an anticancer medicament having cytotoxic properties in timed release form;
wherein the therapeutic composition is released substantially immediately and the anticancer medicament is released after a period of time sufficient such that the cancerous cells have substantially metabolized the therapeutic composition and the non-cancerous cells have not substantially metabolized the therapeutic composition.

In a further embodiment, the components in the therapeutic compositions may be utilized in wound healing products to increase the resuscitation rate of injured mammalian cells. In this embodiment, the mixture of saturated and unsaturated fatty acids is employed with another component in the therapeutic compositions. Not all components of the therapeutic composition may be required in the wound healing product to heal a wound. For example, if the wound is not undergoing oxidative injury, the therapeutic composition may not require an antioxidant. For other types of wounds, the components in the therapeutic composition may vary depending upon the type of wound being treated.

In a specific embodiment, the invention is directed at a wound healing pharmaceutical composition to increase the resuscitation rate of injured mammalian cells which comprises:
(A) a first wound healing component comprising a mixture of saturated and unsaturated fatty acids wherein the fatty acids are those fatty acids required for the resuscitation of injured mammalian cells; and
(B) a second wound healing component selected from the group consisting of:
   (a) pyruvate selected from the group consisting of pyruvic acid, pharmaceutically acceptable salts of pyruvic acid, and mixtures thereof.

The present invention extends to methods for making the augmented pharmaceutical compositions, cytoprotective pharmaceutical compositions, prophylactic cytoprotective pharmaceutical compositions, timed release prophylactic cytoprotective pharmaceutical compositions, preventative cytoprotective pharmaceutical compositions, timed release preventative cytoprotective pharmaceutical compositions, and wound healing pharmaceutical compositions. In general, the pharmaceutical compositions are made by forming an admixture of the therapeutic components in the compositions. For example, an augmented pharmaceutical composition is made by forming an admixture of the therapeutic compositions and the medicament which is useful for treating injured mammalian cells. A cytoprotective pharmaceutical composition is made by forming an admixture of the therapeutic compositions and the medicament having cytotoxic properties. A prophylactic cytoprotective pharmaceutical composition, including the timed release form, is made by forming an admixture of the prophylactic therapeutic components and the medicament having cytotoxic properties. A preventative cytoprotective pharmaceutical composition, including the timed release form, is made by forming an admixture of the preventative therapeutic components and the medicament having cytotoxic properties. A wound healing pharmaceutical composition is made by forming an admixture of the wound healing components.

The present invention also extends to methods for employing the augmented pharmaceutical compositions, the cytoprotective pharmaceutical compositions, the prophylactic cytoprotective pharmaceutical compositions, the timed release prophylactic cytoprotective pharmaceutical compositions, the preventative cytoprotective pharmaceutical compositions, the timed release preventative cytoprotective pharmaceutical compositions, and the wound healing pharmaceutical compositions.

In general, a pharmaceutical composition is employed by contacting the pharmaceutical composition with mammalian cells.

The therapeutic compositions of the present invention may also be administered to cells prior to the administration of a cytotoxic chemotherapeutic medicament, for example 15 minutes prior to administering the chemotherapeutic medicament, to selectively protect non-cancerous cells in the presence of cancerous cells against a cytotoxic chemotherapeutic medicament. Because cancerous cells have a rapid metabolism, cancerous cells will rapidly consume the protective therapeutic composition and will not be protected by the therapeutic compositions when the chemotherapeutic medicament is subsequently administered.

The types of wounds which may be healed using the compositions of the present invention are those which result from an injury which causes epidermal damage, chronic ulcers, gastric ulcers, burns, and donor site wounds. Such wounds include ophthalmic wounds, such as those which result from corneal ulcers, radialkeratotomy, corneal transplants, epikeratophakia and other surgically induced wounds in the eye, and cutaneous wounds such as burn wounds, donor site wounds from skin transplants and ulcers (cutaneous, decubitis, venous stasis, and diabetic). In addition, dermatological wounds such as psoriasis, sunburn, and skin rashes may also be treated with the compositions of the present invention. The compositions may be applied to the wound site either topically or internally depending on the type of wound.

The composition is maintained in contact with the wound for a period of time sufficient to increase the rate of cell growth at the wound site.

In another embodiment, the invention is directed at a method for preserving mammalian cells in a culture medium which comprises the steps of:
(A) providing a therapeutic composition selected from the group of consisting of:
   (1)
      (a) pyruvate selected from the group consisting of pyruvic acid, pharmaceutically acceptable salts of pyruvic acid, and mixtures thereof;
      (b) an antioxidant; and
      (c) a mixture of saturated and unsaturated fatty acids wherein the fatty acids are those fatty acids required for the resuscitation of injured mammalian cells;
(B) providing mammalian cells in a culture medium; and
(C) contacting the therapeutic composition from step (A) with the mammalian cells in the culture medium from step (B).

Once prepared, the inventive therapeutic compositions may be stored for future use or may be formulated in effective amounts with pharmaceutically acceptable carriers to prepare a wide variety of pharmaceutical compositions. Examples of pharmaceutically acceptable carriers are pharmaceutical appliances, topical vehicles (non-oral and oral), and ingestible vehicles.

Examples of pharmaceutical appliances are sutures, staples, gauze, bandages, burn dressings, artificial skins, liposome or micell formulations, microcapsules, aqueous vehicles for soaking gauze dressings, and the like, and mixtures thereof. Non-oral topical compositions employ non-oral topical vehicles, such as creams, gels formulations, foams, ointments and sprays, salves, and films, which are intended to be applied to the skin or body cavity and are not intended to be taken by mouth. Oral topical compositions employ oral vehicles, such as mouthwashes, rinses, oral sprays, suspensions, and dental gels, which are intended to be taken by mouth but are not intended to be ingested. Ingestible compositions employ ingestible or partly ingestible vehicles such as confectionery bulking agents which include hard and soft confectionery such as lozenges, tablets, toffees, nougats, suspensions, chewy candies, and chewing gums.

In one form of the invention, the therapeutic composition is incorporated into a pharmaceutical appliance which may be in the form of sutures, staples, gauze, bandages, burn dressings, artificial skins, liposome or micell formulations, microcapsules, aqueous vehicles for soaking gauze dressings, and the like, and mixtures thereof. A variety of traditional ingredients may optionally be included in the pharmaceutical composition in effective amounts such as buffers, preservatives, tonicity adjusting agents, antioxidants, polymers for adjusting viscosity or for use as extenders, and excipients, and the like. Specific illustrative examples of such traditional ingredients include acetate and borate buffers; thimerosol, sorbic acid, methyl and propyl paraben and chlorobutanol preservatives; sodium chloride and sugars to adjust the tonicity; and excipients such as mannitol, lactose and sucrose. Other conventional pharmaceutical additives known to those having ordinary skill in the pharmaceutical arts may also be used in the pharmaceutical composition.

In accordance with this invention, therapeutically effective amounts of the therapeutic compositions of the present invention may be employed in the pharmaceutical appliance. These amounts are readily determined by those skilled in the art without the need for undue experimentation. The exact amount of the therapeutic composition employed is subject to such factors as the type and concentration of the therapeutic composition and the type of pharmaceutical appliance employed. Thus, the amount of therapeutic composition may be varied in order to obtain the result desired in the final product and such variations are within the capabilities of those skilled in the art without the need for undue experimentation. In a preferred embodiment, the pharmaceutical composition will comprise the therapeutic composition in an amount from about 0.1% to about 5%, by weight of the pharmaceutical composition. In a more preferred embodiment, the pharmaceutical composition will comprise the therapeutic composition in an amount from about 0.1% to about 3%, by weight of the pharmaceutical composition. In a most preferred embodiment, the pharmaceutical composition will comprise the therapeutic composition in an amount from about 0.1% to about 1%, by weight of the pharmaceutical composition.

The present invention extends to methods for making the pharmaceutical compositions. In general, a pharmaceutical composition is made by contacting a therapeutically effective amount of a therapeutic composition with a pharmaceutical appliance and the other ingredients of the final desired pharmaceutical composition. The therapeutic composition may be in a solvent and may be absorbed onto a pharmaceutical appliance.

Other ingredients will usually be incorporated into the composition as dictated by the nature of the desired composition as well known by those having ordinary skill in the art. The ultimate pharmaceutical compositions are readily prepared using methods generally known in the pharmaceutical arts.

In another form of the invention, the therapeutic composition is incorporated into a non-oral topical vehicle which may be in the form of a cream, gel, foam, ointment, spray, and the like. Typical non-toxic non-oral topical vehicles known in the pharmaceutical arts may be used in the present invention. The preferred non-oral topical vehicles are water and pharmaceutically acceptable water-miscible organic solvents such as ethyl alcohol, isopropyl alcohol, propylene glycol, glycerin, and the like, and mixtures of these solvents. Water-alcohol mixtures are particularly preferred and are generally employed in a weight ratio from about 1:1 to about 20:1, preferably from about 3:1 to about 20:1, and most preferably from about 3:1 to about 10:1, respectively.

The non-oral topical therapeutic compositions may also contain conventional additives employed in those products. Conventional additives include humectants, emollients, lubricants, stabilizers, dyes, and perfumes, providing the additives do not interfere with the therapeutic properties of the therapeutic composition.

Suitable humectants useful in the non-oral topical therapeutic compositions include glycerin, propylene glycol, polyethylene glycol, sorbitan, fructose, and the like, and mixtures thereof. Humectants, when employed, may be present in amounts from about 10% to about 20%, by weight of the topical therapeutic composition.

The coloring agents (colors, colorants) useful in the non-oral topical therapeutic composition are used in amounts effective to produce the desired color. These coloring agents include pigments which may be incorporated in amounts up to about 6% by weight of the non-oral topical therapeutic composition. A preferred pigment, titanium dioxide, may be incorporated in amounts up to about 2%, and preferably less than about 1%, by weight of the non-oral topical therapeutic composition. The coloring agents may also include natural food colors and dyes suitable for food, drug and cosmetic applications. These coloring agents are known as F.D.& C. dyes and lakes. The materials acceptable for the foregoing uses are preferably water-soluble. Illustrative nonlimiting examples include the indigoid dye known as F.D.& C. Blue No.2, which is the disodium salt of 5,5-indigotindisulfonic acid. Similarly, the dye known as F.D.& C. Green No.1 comprises a triphenylmethane dye and is the monosodium salt of 4-[4-(N-ethyl-p-sulfoniumbenzylamino) diphenylmethylene]-[1-(N-ethyl-N-p-sulfoniumbenzyl)-delta-2,5-cyclohexadieneimine]. A full recitation of all F.D.& C. coloring agents and their corresponding chemical structures may be found in the Kirk-Othmer Encyclopedia of Chemical Technology, 3rd Edition, in volume 5 at pages 857-884, which text is incorporated herein by reference.

In accordance with this invention, therapeutically effective amounts of the therapeutic compositions of the present invention may be admixed with a non-oral topical vehicle to form a topical therapeutic composition. These amounts are readily determined by those skilled in the art without the need for undue experimentation. In a preferred embodiment, the non-oral topical therapeutic compositions will comprise the therapeutic composition in an amount from about 0.1% to about 10% and a non-oral topical vehicle in a quantity sufficient to bring the total amount of composition to 100%, by weight of the non-oral topical therapeutic composition. In a more preferred embodiment, the non-oral topical therapeutic compositions will comprise the therapeutic composition in an amount from about 0.1% to about 5%, and in a most preferred embodiment, the non-oral topical therapeutic compositions will comprise the therapeutic composition in an amount from about 0.1% to about 2%, and a non-oral topical vehicle in a quantity sufficient to bring the total amount of composition to 100%, by weight of the non-oral topical therapeutic composition.

The present invention extends to methods for preparing the non-oral topical therapeutic compositions. In such a method, the non-oral topical therapeutic composition is prepared by admixing a therapeutically effective amount of the therapeutic composition of the present invention and a non-oral topical vehicle. The final compositions are readily prepared using standard methods and apparatus generally known by those skilled in the pharmaceutical arts. The apparatus useful in accordance with the present invention comprises mixing apparatus well known in the pharmaceutical arts, and therefore the selection of the specific apparatus will be apparent to the artisan.

In another form of the invention, the therapeutic composition is incorporated into an oral topical vehicle which may be in the form of a mouthwash, rinse, oral spray, suspension, dental gel, and the like. Typical non-toxic oral vehicles known in the pharmaceutical arts may be used in the present invention. The preferred oral vehicles are water, ethanol, and water-ethanol mixtures. The water-ethanol mixtures are generally employed in a weight ratio from about 1:1 to about 20:1, preferably from about 3:1 to about 20:1, and most preferably from about 3:1 to about 10:1, respectively. The pH value of the oral vehicle is generally from about 4 to about 7, and preferably from about 5 to about 6.5. An oral topical vehicle having a pH value below about 4 is generally irritating to the oral cavity and an oral vehicle having a pH value greater than about 7 generally results in an unpleasant mouth feel.

The oral topical therapeutic compositions may also contain conventional additives normally employed in those products. Conventional additives include a fluorine providing compound, a sweetening agent, a flavoring agent, a coloring agent, a humectant, a buffer, and an emulsifier, providing the additives do not interfere with the therapeutic properties of the therapeutic composition.

The coloring agents and humectants, and the amounts of these additives to be employed, set out above as useful in the non-oral topical therapeutic composition may be used in the oral topical therapeutic composition.

Fluorine providing compounds may be fully or slightly water soluble and are characterized by their ability to release fluoride ions or fluoride containing ions in water and by their lack of reaction with other components in the composition. Typical fluorine providing compounds are inorganic fluoride salts such as water-soluble alkali metal, alkaline earth metal, and heavy metal salts, for example, sodium fluoride, potassium fluoride, ammonium fluoride, cuprous fluoride, zinc fluoride, stannic fluoride, stannous fluoride, barium fluoride, sodium fluorosilicate, ammonium fluorosilicate, sodium fluorozirconate, sodium monofluorophosphate, aluminum mono- and difluorophosphates and fluorinated sodium calcium pyrophosphate. Alkali metal fluorides, tin fluoride and monofluorophosphates, such as sodium and stannous fluoride, sodium monofluorophosphate and mixtures thereof, are preferred.

The amount of fluorine providing compound present in the present oral topical therapeutic composition is dependent upon the type of fluorine providing compound employed, the solubility of the fluorine compound, and the nature of the final oral therapeutic composition. The amount of fluorine providing compound used must be a nontoxic amount. In general, the fluorine providing compound when used will be present in an amount up to about 1%, preferably from about 0.001% to about 0.1%, and most preferably from about 0.001% to about 0.05%, by weight of the oral topical therapeutic composition.

When sweetening agents (sweeteners) are used, those sweeteners well known in the art, including both natural and artificial sweeteners, may be employed. The sweetening agent used may be selected from a wide range of materials including water-soluble sweetening agents, water-soluble artificial sweetening agents, water-soluble sweetening agents derived from naturally occurring water-soluble sweetening agents, dipeptide based sweetening agents, and protein based sweetening agents, including mixtures thereof. Without being limited to particular sweetening agents, representative categories and examples include:
(a) water-soluble sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin, and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and the like;
(c) dipeptide based sweeteners, such as L-aspartic acid derived sweeteners, such as L-aspartyl-L-phenylalanine methyl ester (Aspartame) and materials described in United States patent no. 3,492,131, L-alpha-aspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenylglycine, L-aspartyl-2,5-dihydro-L-phenylalanine; L-aspartyl-L-(1-cyclohexen)-alanine, and the like;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-furanoside, or 4,1'-dichloro-4,1'-dideoxygalactosucrose; 1',6'-dichloro-1',6'-dideoxysucrose; 4-chloro-4-deoxy-alpha-D-galactopyranosyl-1,6-dichloro-1,6-dideoxy-beta-D-fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D-fructofuranoside, or 4,6,6,-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideoxy-beta-D-fructofuranoside, or 4,6,1',6'-tetrachloro-4,6,1',6'-tetradeoxygalacto-sucrose; and 4,6,1',6'-tetrachloro-4,6,1',6'-tetradeoxy-sucrose; and
(e) protein based sweeteners such as thaumaoccous danielli (Thaumatin I and II).

In general, an effective amount of sweetening agent is utilized to provide the level of sweetness desired in the particular oral topical therapeutic composition, and this amount will vary with the sweetener selected and the final oral therapeutic product desired. The amount of sweetener normally present is in the range from about 0.0025% to about 90%, by weight of the oral topical therapeutic composition, depending upon the sweetener used. The exact range of amounts for each type of sweetener is well known in the art and is not the subject of the present invention.

The flavoring agents (flavors, flavorants) which may be used include those flavors known to the skilled artisan, such as natural and artificial flavors. Suitable flavoring agents include mints, such as peppermint, citrus flavors such as orange and lemon, artificial vanilla, cinnamon, various fruit flavors, both individual and mixed, and the like.

The amount of flavoring agent employed in the oral topical therapeutic composition is normally a matter of preference subject to such factors as the type of final oral therapeutic composition, the individual flavor employed, and the strength of flavor desired. Thus, the amount of flavoring may be varied in order to obtain the result desired in the final product and such variations are within the capabilities of those skilled in the art without the need for undue experimentation. The flavoring agents, when used, are generally utilized in amounts that may, for example, range in amounts from about 0.05% to about 6%, by weight of the oral topical therapeutic composition.

Suitable buffer solutions useful in the non-oral topical therapeutic compositions include citric acid-sodium citrate solution, phosphoric acid-sodium phosphate solution, and acetic acid-sodium acetate solution in amounts up to about 1%, and preferably from about 0.05% to about 0.5% by weight of the oral topical therapeutic composition.

In accordance with this invention, therapeutically effective amounts of the therapeutic compositions of the present invention may be admixed with an oral topical vehicle to form a topical therapeutic composition. These amounts are readily determined by those skilled in the art without the need for undue experimentation. In a preferred embodiment, the oral topical therapeutic compositions will comprise the therapeutic composition in an amount from about 0.1% to about 10% and a oral topical vehicle in a quantity sufficient to bring the total amount of composition to 100%, by weight of the oral topical therapeutic composition. In a more preferred embodiment, the oral topical therapeutic compositions will comprise the therapeutic composition in an amount from about 0.1% to about 5%, and in a most preferred embodiment, the oral topical therapeutic compositions will comprise the therapeutic composition in an amount from about 0.1% to about 2%, and a oral topical vehicle in a quantity sufficient to bring the total amount of composition to 100%, by weight of the oral topical therapeutic composition.

The present invention extends to methods for preparing the oral topical therapeutic compositions. In such a method, the oral topical therapeutic composition is prepared by admixing a therapeutically effective amount of the therapeutic composition of the present invention and an oral topical vehicle. The final compositions are readily prepared using standard methods and apparatus generally known by those skilled in the pharmaceutical arts. The apparatus useful in accordance with the present invention comprises mixing apparatus well known in the pharmaceutical arts, and therefore the selection of the specific apparatus will be apparent to the artisan.

In a preferred embodiment, an oral topical therapeutic composition is made by first dissolving coloring agents, sweetening agents, and similar additives in water. The therapeutic composition is then admixed with the aqueous solution. Then sufficient water or ethanol, or mixtures of water and ethanol, are added to the solution with mixing until the final solution volume is reached. In a more preferred embodiment, the therapeutic composition is added to the solution as the final ingredient. The final oral topical therapeutic compositions are readily prepared using methods generally known in the pharmaceutical arts.

The oral therapeutic composition may also be in the form of dental gel. As used herein, the term "gel" means a solid or semisolid colloid which contains considerable quantities of water. The colloid particles in a gel are linked together in a coherent meshwork which immobilizes the water contained inside the meshwork.

The dental gel compositions of the present invention may contain the conventional additives set out above for oral topical therapeutic compositions such as mouthwashes, rinses, oral sprays, and suspensions and, in addition, may contain additional additives such as a polishing agent, a desensitizing agent, and the like, providing the additional additives do not interfere with the therapeutic properties of the therapeutic composition.

In a dental gel composition, the oral vehicle generally comprises water, typically in an amount from about 10% to about 90%, by weight of the dental gel composition. Polyethylene glycol, propylene glycol, glycerin, and mixtures thereof may also be present in the vehicle as humectants or binders in amounts from about 18% to about 30%, by weight of the dental gel composition. Particularly preferred oral vehicles comprise mixtures of water with polyethylene glycol or water with glycerin and polypropylene glycol.

The dental gels of the present invention include a gelling agent (thickening agent) such as a natural or synthetic gum or gelatin. Gelling agents such as hydroxyethyl cellulose, methyl cellulose, glycerin, carboxypolymethylene, and gelatin and the like, and mixtures thereof may be used. The preferred gelling agent is hydroxyethyl cellulose. Gelling agents may be used in amounts from about 0.5% to about 5%, and preferably from about 0.5% to about 2%, by weight of the dental gel composition.

The dental gel compositions of the present invention may also include a polishing agent. In clear gels, a polishing agent of colloidal silica and/or alkali metal aluminosilicate complexes is preferred since these materials have refractive indices close to the refractive indices of the gelling systems commonly used in dental gels. In non-clear gels, a polishing agent of calcium carbonate or calcium dihydrate may be used. These polishing agents may be used in amounts up to about 75%, and preferably in amounts up to about 50%, by weight of the dental gel composition.

The dental gel may also contain a desensitizing agent such as a combination of citric acid and sodium citrate. Citric acid may be used in an amount from about 0.1% to about 3%, and preferably from about 0.2% to about 1%, by weight, and sodium citrate may be used in an amount from about 0.3% to about 9%, and preferably from about 0.6% to about 3%, by weight of the dental gel composition.

In accordance with this invention, therapeutically effective amounts of the therapeutic compositions of the present invention may be admixed into the dental gel compositions. These amounts are readily determined by those skilled in the art without the need for undue experimentation. In a preferred embodiment, the dental gel compositions will comprise the therapeutic composition in an amount from about 0.1% to about 10% and an oral topical vehicle in a quantity sufficient to bring the total amount of composition to 100%, by weight of the dental gel composition. In a more preferred embodiment, the dental gel compositions will comprise the therapeutic composition in an amount from about 0.1% to about 5%, and in a most preferred embodiment, the dental gel compositions will comprise the therapeutic composition in an amount from about 0.1% to about 2%, and an oral topical vehicle in a quantity sufficient to bring the total amount of composition to 100%, by weight of the dental gel composition.

The present invention extends to methods for preparing the therapeutic dental gel compositions. In such a method, the dental gel composition is prepared by admixing a therapeutically effective amount of the therapeutic composition of the present invention and an oral topical vehicle. The final compositions are readily prepared using methods generally known by those skilled in the dental and pharmaceutical arts. The apparatus useful in accordance with the present invention comprises mixing apparatus well known in the pharmaceutical arts, and therefore the selection of the specific apparatus will be apparent to the artisan.

In a preferred embodiment, a therapeutic dental gel composition is made by first dispersing a gelling agent in a humectant or water, or a mixture of both, then admixing to the dispersion an aqueous solution of the water-soluble additives such as the fluorine providing compound, sweeteners and the like, then adding the polishing agent, and lastly admixing the flavoring agent and the therapeutic composition. The final gel mixture is then tubed or otherwise packaged. The liquids and solids in a gel product are proportioned to form a creamy or gelled mass which is extrudable from a pressurized container or from a collapsible tube. The final therapeutic compositions are readily prepared using methods generally known in the pharmaceutical arts.

In yet another form of the invention, the therapeutic composition is incorporated into an ingestible vehicle. The ingestible vehicle may be a confectionery bulking agent in the form of lozenges, tablets, toffees, nougats, suspensions, chewy candies, chewing gums, and the like. The pharmaceutically acceptable carriers may be prepared from a wide range of materials including, but not limited to, diluents, binders and adhesives, lubricants, disintegrants, coloring agents, bulking agents, flavoring agents, sweetening agents and miscellaneous materials such as buffers and adsorbents that may be needed in order to prepare a particular therapeutic confection.

The preparation of confectionery formulations is historically well known and has changed little through the years. Confectionery items have been classified as either "hard" confectionery or "soft" confectionery. The therapeutic compositions of the present invention can be incorporated into confectionery compositions by admixing the inventive composition into conventional hard and soft confections.

As used herein, the term confectionery material means a product containing a bulking agent selected from a wide variety of materials such as sugar, corn syrup, and in the case of sugarless bulking agents, sugar alcohols such as sorbitol and mannitol and mixtures thereof. Confectionery material may include such exemplary substances as lozenges, tablets, toffee, nougat, suspensions, chewy candy, chewing gum and the like. The bulking agent is present in a quantity sufficient to bring the total amount of composition to 100%. In general, the bulking agent will be present in amounts up to about 99.98%, preferably in amounts up to about 99.9%, and more preferably in amounts up to about 99%, by weight of the ingestible therapeutic composition.

Lozenges are flavored medicated dosage forms intended to be sucked and held in the mouth. Lozenges may be in the form of various shapes such as flat, circular, octagonal and biconvex forms. The lozenge bases are generally in two forms: hard boiled candy lozenges and compressed tablet lozenges.

Hard boiled candy lozenges may be processed and formulated by conventional means. In general, a hard boiled candy lozenge has a base composed of a mixture of sugar and other carbohydrate bulking agents kept in an amorphous or glassy condition. This amorphous or glassy form is considered a solid syrup of sugars generally having from about 0.5% to about 1.5% moisture. Such materials normally contain up to about 92% corn syrup, up to about 55% sugar and from about 0.1% to about 5% water, by weight of the final composition. The syrup component is generally prepared from corn syrups high in fructose, but may include other materials. Further ingredients such as flavoring agents, sweetening agents, acidulants, coloring agents and the like may also be added.

Boiled candy lozenges may also be prepared from non-fermentable sugars such as sorbitol, mannitol, and hydrogenated corn syrup. Typical hydrogenated corn syrups are Lycasin, a commercially available product manufactured by Roquette Corporation, and Hystar, a commercially available product manufactured by Lonza, Inc. The candy lozenges may contain up to about 95% sorbitol, a mixture of sorbitol and mannitol in a ratio from about 9.5:0.5 up to about 7.5:2.5, and hydrogenated corn syrup up to about 55%, by weight of the solid syrup component.

Boiled candy lozenges may be routinely prepared by conventional methods such as those involving fire cookers, vacuum cookers, and scraped-surface cookers also referred to as high speed atmospheric cookers.

Fire cookers involve the traditional method of making a boiled candy lozenge base. In this method, the desired quantity of carbohydrate bulking agent is dissolved in water by heating the agent in a kettle until the bulking agent dissolves. Additional bulking agent may then be added and cooking continued until a final temperature of 145^{o} C. to 156^{o} C. is achieved. The batch is then cooled and worked as a plastic-like mass to incorporate additives such as flavors, colorants and the like.

A high-speed atmospheric cooker uses a heat-exchanger surface which involves spreading a film of candy on a heat exchange surface, the candy is heated to 165^{o} C. to 170^{o} C. in a few minutes. The candy is then rapidly cooled to 100^{o} C. to 120^{o} C. and worked as a plastic-like mass enabling incorporation of the additives, such as flavors, colorants and the like.

In vacuum cookers, the carbohydrate bulking agent is boiled to 125^{o} C. to 132^{o} C., vacuum is applied and additional water is boiled off without extra heating. When cooking is complete, the mass is a semi-solid and has a plastic-like consistency. At this point, flavors, colorants, and other additives are admixed in the mass by routine mechanical mixing operations.

The optimum mixing required to uniformly mix the flavoring agents, coloring agents and other additives during conventional manufacturing of boiled candy lozenges is determined by the time needed to obtain a uniform distribution of the materials. Normally, mixing times of from 4 to 10 minutes have been found to be acceptable.

Once the boiled candy lozenge has been properly tempered, it may be cut into workable portions or formed into desired shapes. A variety of forming techniques may be utilized depending upon the shape and size of the final product desired. A general discussion of the composition and preparation of hard confections may be found in H.A. Lieberman, Pharmaceutical Dosage Forms: Tablets, Volume 1 (1980), Marcel Dekker, Inc., New York, N.Y. at pages 339 to 469, which disclosure is incorporated herein by reference.

The apparatus useful in accordance with the present invention comprises cooking and mixing apparatus well known in the confectionery manufacturing arts, and therefore the selection of the specific apparatus will be apparent to the artisan.

In contrast, compressed tablet confections contain particulate materials and are formed into structures under pressure. These confections generally contain sugars in amounts up to about 95%, by weight of the composition, and typical tablet excipients such as binders and lubricants as well as flavoring agents, coloring agents and the like.

In addition to hard confectionery materials, the lozenges of the present invention may be made of soft confectionery materials such as those contained in nougat. The preparation of soft confections, such as nougat, involves conventional methods, such as the combination of two primary components, namely (1) a high boiling syrup such as a corn syrup, hydrogenated starch hydrolysate or the like, and (2) a relatively light textured frappe, generally prepared from egg albumin, gelatin, vegetable proteins, such as soy derived compounds, sugarless milk derived compounds such as milk proteins, and mixtures thereof. The frappe is generally relatively light, and may, for example, range in density from about 0.5 to about 0.7 grams/cc.

The high boiling syrup, or "bob syrup" of the soft confectionery is relatively viscous and has a higher density than the frappe component, and frequently contains a substantial amount of carbohydrate bulking agent such as a hydrogenated starch hydrolysate. Conventionally, the final nougat composition is prepared by the addition of the "bob syrup" to the frappe under agitation, to form the basic nougat mixture. Further ingredients such as flavoring agents, additional carbohydrate bulking agent, coloring agents, preservatives, medicaments, mixtures thereof and the like may be added thereafter also under agitation. A general discussion of the composition and preparation of nougat confections may be found in B.W. Minifie, Chocolate, Cocoa and Confectionery: Science and Technology, 2nd edition, AVI Publishing Co., Inc., Westport, Conn. (1980), at pages 424-425, which disclosure is incorporated herein by reference.

The procedure for preparing the soft confectionery involves known procedures. In general, the frappe component is prepared first and thereafter the syrup component is slowly added under agitation at a temperature of at least about 65^{o} C., and preferably at least about 100^{o} C. The mixture of components is continued to be mixed to form a uniform mixture, after which the mixture is cooled to a temperature below 80^{o} C., at which point, the flavoring agent may be added. The mixture is further mixed for an additional period until it is ready to be removed and formed into suitable confectionery shapes.

The ingestible therapeutic compositions may also be in the form of a pharmaceutical suspension. Pharmaceutical suspensions of this invention may be prepared by conventional methods long established in the art of pharmaceutical compounding. Suspensions may contain adjunct materials employed in formulating the suspensions of the art. The suspensions of the present invention can comprise:
(a) preservatives such as butylated hydroxyanisole (BHA), butylated hydroxytoluene (BHT), benzoic acid, ascorbic acid, methyl paraben, propyl paraben, tocopherols, and the like, and mixtures thereof. Preservatives are generally present in amounts up to about 1%, and preferably from about 0.05% to about 0.5%, by weight of the suspension;
(b) buffers such as citric acid-sodium citrate, phosphoric acid-sodium phosphate, and acetic acid-sodium acetate in amounts up to about 1%, and preferably from about 0.05% to about 0.5%, by weight of the suspension;
(c) suspending agents or thickeners such as cellulosics like methylcellulose, carrageenans like alginic acid and its derivatives, xanthan gums, gelatin, acacis, and microcrystalline cellulose in amounts up to about 20%, and preferably from about 1% to about 15%, by weight of the suspension;
(d) antifoaming agents such as dimethyl polysiloxane in amounts up to about 0.2%, and preferably from about 0.01% to about 0.1%, by weight of the suspension;
(e) sweetening agents such as those sweeteners well known in the art, including both natural and artificial sweeteners. Sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, glycyrrhizin, and sugar alcohols such as sorbitol, mannitol, maltitol, hydrogenated starch hydrolysates and mixtures thereof may be utilized in amounts up to about 60%, and preferably from about 20% to about 50%, by weight of the suspension. Water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and the like may be utilized in amounts from about 0.001% to about 5%, by weight of the suspension;
(f) flavoring agents such as those flavors well known to the skilled artisan, such as natural and artificial flavors and mints, such as peppermint, menthol, citrus flavors such as orange and lemon, artificial vanilla, cinnamon, various fruit flavors, both individual and mixed and the like may be utilized in amounts from about 0.5% to about 5%, by weight of the suspension;
(g) coloring agents such as pigments which may be incorporated in amounts up to about 6%, by weight of the suspension. A preferred pigment, titanium dioxide, may be incorporated in amounts up to about 2%, and preferably less than about 1%, by weight of the suspension. The coloring agents may also include natural food colors and dyes suitable for food, drug and cosmetic applications. These colorants are known as F.D.& C. dyes and lakes. The materials acceptable for the foregoing uses are preferably water-soluble. Such dyes are generally present in amounts up to about 0.25%, and preferably from about 0.05% to about 0.2%, by weight of the suspension;
(h) decolorizing agents such as sodium metabisulfite, ascorbic acid and the like may be incorporated into the suspension to prevent color changes due to aging. In general, decolorizing agents may be used in amounts up to about 0.25%, and preferably from about 0.05% to about 0.2%, by weight of the suspension; and
(i) solubilizers such as alcohol, propylene glycol, polyethylene glycol, and the like may be used to solubilize the flavoring agents. In general, solubilizing agents may be used in amounts up to about 10%, and preferably from about 2% to about 5%, by weight of the suspension.

The pharmaceutical suspensions of the present invention may be prepared as follows:
(A) admix the thickener with water heated from about 40^{o} C. to about 95^{o} C., preferably from about 40^{o} C. to about 70^{o} C., to form a dispersion if the thickener is not water soluble or a solution if the thickener is water soluble;
(B) admix the sweetening agent with water to form a solution;
(C) admix the therapeutic composition with the thickener-water admixture to form a uniform thickener-therapeutic composition;
(D) combine the sweetener solution with the thickener-therapeutic composition and mix until uniform; and
(E) admix the optional adjunct materials such as coloring agents, flavoring agents, decolorants, solubilizers, antifoaming agents, buffers and additional water with the mixture of step (D) to form the suspension.

The ingestible therapeutic compositions of this invention may also be in chewable form. To achieve acceptable stability and quality as well as good taste and mouth feel in a chewable formulation several considerations are important. These considerations include the amount of active substance per tablet, the flavoring agent employed, the degree of compressibility of the tablet and the organoleptic properties of the composition.

Chewable therapeutic candy is prepared by procedures similar to those used to make soft confectionery. In a typical procedure, a boiled sugar-corn syrup blend is formed to which is added a frappe mixture. The boiled sugar-corn syrup blend may be prepared from sugar and corn syrup blended in parts by weight ratio of about 90:10 to about 10:90. The sugar-corn syrup blend is heated to temperatures above about 120^{o} C. to remove water and to form a molten mass. The frappe is generally prepared from gelatin, egg albumin, milk proteins such as casein, and vegetable proteins such as soy protein, and the like, which is added to a gelatin solution and rapidly mixed at ambient temperature to form an aerated sponge like mass. The frappe is then added to the molten candy mass and mixed until homogeneous at temperatures between about 65^{o} C. and about 120^{o} C.

The ingestible therapeutic composition of the instant invention can then be added to the homogeneous mixture as the temperature is lowered to about 65^{o} C.-95^{o} C. whereupon additional ingredients can then be added such as flavoring agents and coloring agents. The formulation is further cooled and formed into pieces of desired dimensions.

A general discussion of the lozenge and chewable tablet forms of confectionery may be found in H.A. Lieberman and L. Lachman, Pharmaceutical Dosage Forms: Tablets Volume 1, Marcel Dekker, Inc., New York, N.Y. at pages 289 to 466, which disclosure is incorporated herein by reference.

In accordance with this invention, therapeutically effective amounts of the therapeutic compositions of the present invention may be admixed into the hard and soft confectionery products. These amounts are readily determined by those skilled in the art without the need for undue experimentation. In a preferred embodiment, the ingestible therapeutic composition will comprise the therapeutic composition in an amount from about 0.1% to about 10% and an ingestible vehicle, that is a pharmaceutically acceptable carrier, in a quantity sufficient to bring the total amount of composition to 100%, by weight the ingestible therapeutic composition. In a more preferred embodiment, the ingestible composition will comprise the therapeutic composition in an amount from about 0.1% to about 5%, and in a most preferred embodiment, the ingestible composition will comprise the therapeutic composition in an amount from about 0.1% to about 2%, and an ingestible vehicle in a quantity sufficient to bring the total amount of composition to 100%, by weight the ingestible therapeutic composition.

The present invention extends to methods of making the ingestible therapeutic compositions. In such methods, an ingestible therapeutic composition is prepared by admixing a therapeutically effective amount of the therapeutic composition with a pharmaceutically-acceptable carrier. The apparatus useful in accordance with the present invention comprises mixing and heating apparatus well known in the confectionery arts, and therefore the selection of the specific apparatus will be apparent to the artisan. The final ingestible therapeutic compositions are readily prepared using methods generally known in the confectionery arts.

The therapeutic compositions may also be incorporated into chewing gums. In this form of the invention, the chewing gum composition contains a gum base, a bulking agent, the inventive therapeutic composition, and various additives.

The gum base employed will vary greatly depending upon various factors such as the type of base desired, the consistency of gum desired and the other components used in the composition to make the final chewing gum product. The gum base may be any water-insoluble gum base known in the art, and includes those gum bases utilized for chewing gums and bubble gums. Illustrative examples of suitable polymers in gum bases include both natural and synthetic elastomers and rubbers. For example, those polymers which are suitable as gum bases include, without limitation, substances of vegetable origin such as chicle, crown gum, nispero, rosadinha, jelutong, perillo, niger gutta, tunu, balata, gutta-percha, lechi-capsi, sorva, gutta kay, mixtures thereof and the like. Synthetic elastomers such as butadiene-styrene copolymers, polyisobutylene, isobutylene-isoprene copolymers, polyethylene, mixtures thereof and the like are particularly useful.

The gum base may include a non-toxic vinyl polymer, such as polyvinyl acetate and its partial hydrolysate, polyvinyl alcohol, and mixtures thereof. When utilized, the molecular weight of the vinyl polymer may range from about 2,000 up to and including about 94,000.

The amount of gum base employed will vary greatly depending upon various factors such as the type of base used, the consistency of the gum desired and the other components used in the composition to make the final chewing gum product. In general, the gum base will be present in amounts from about 5% to about 94%, by weight of the final chewing gum composition, and preferably in amounts from about 15% to about 45%, and more preferably in amounts from about 15% to about 35%, and most preferably in amounts from about 20% to about 30%, by weight of the final chewing gum composition.

The gum base composition may contain conventional elastomer solvents to aid in softening the elastomer base component. Such elastomer solvents may comprise terpinene resins such as polymers of alpha-pinene or beta-pinene, methyl, glycerol or pentaerythritol esters of rosins or modified rosins and gums, such as hydrogenated, dimerized or polymerized rosins or mixtures thereof. Examples of elastomer solvents suitable for use herein include the pentaerythritol ester of partially hydrogenated wood or gum rosin, the pentaerythritol ester of wood or gum rosin, the glycerol ester of wood rosin, the glycerol ester of partially dimerized wood or gum rosin, the glycerol ester of polymerized wood or gum rosin, the glycerol ester of tall oil rosin, the glycerol ester of wood or gum rosin and the partially hydrogenated wood or gum rosin and the partially hydrogenated methyl ester of wood or rosin, mixtures thereof, and the like. The elastomer solvent may be employed in amounts from about 5% to about 75%, by weight of the gum base, and preferably from about 45% to about 70%, by weight of the gum base.

A variety of traditional ingredients may be included in the gum base in effective amounts such as plasticizers or softeners such as lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, mixtures thereof, and the like may also be incorporated into the gum base to obtain a variety of desirable textures and consistency properties. Waxes, for example, natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like may also be incorporated into the gum base to obtain a variety of desirable textures and consistency properties. These traditional additional materials are generally employed in amounts up to about 30%, by weight of the gum base, and preferably in amounts from about 3% to about 20%, by weight of the gum base.

The gum base may include effective amounts of mineral adjuvants such as calcium carbonate, magnesium carbonate, alumina, aluminum hydroxide, aluminum silicate, talc, tricalcium phosphate, dicalcium phosphate and the like as well as mixtures thereof. These mineral adjuvants may serve as fillers and textural agents. These fillers or adjuvants may be used in the gum base in various amounts. Preferably the amount of filler when used will be present in an amount up to about 60%, by weight of the chewing gum base.

The chewing gum base may additionally include the conventional additives of coloring agents, antioxidants, preservatives and the like. For example, titanium dioxide and other dyes suitable for food, drug and cosmetic applications, known as F.D. & C. dyes, may be utilized. An antioxidant such as butylated hydroxytoluene (BHT), butylated hydroxyanisole (BHA), propyl gallate, and mixtures thereof, may also be included. Other conventional chewing gum additives known to one having ordinary skill in the chewing gum art may also be used in the chewing gum base.

The gum composition may include effective amounts of conventional additives selected from the group consisting of sweetening agents (sweeteners), plasticizers, softeners, emulsifiers, waxes, fillers, bulking agents, mineral adjuvants, flavoring agents (flavors, flavorings), coloring agents (colorants, colorings), antioxidants, acidulants, thickeners, mixtures thereof and the like. Some of these additives may serve more than one purpose. For example, in sugarless gum compositions, the sweetener, e.g., sorbitol or other sugar alcohol or mixtures thereof, may also function as a bulking agent. Similarly, in sugar containing gum compositions, the sugar sweetener can also function as a bulking agent.

The plasticizers, softeners, mineral adjuvants, colorants, waxes and antioxidants discussed above as being suitable for use in the gum base may also be used in the gum composition. Examples of other conventional additives which may be used include emulsifiers, such as lecithin and glyceryl monostearate, thickeners, used alone or in combination with other softeners, such as methyl cellulose, alginates, carrageenan, xanthan gum, gelatin, carob, tragacanth, locust bean, and carboxy methyl cellulose, acidulants such as malic acid, adipic acid, citric acid, tartaric acid, fumaric acid, and mixtures thereof, and fillers, such as those discussed above under the category of mineral adjuvants. The fillers when used may be utilized in an amount up to about 60%, by weight of the gum composition.

Bulking agents (carriers, extenders) suitable for use in chewing gums include sweetening agents selected from the group consisting of monosaccharides, disaccharides, poly-saccharides, sugar alcohols, and mixtures thereof; polydextrose; maltodextrins; minerals, such as calcium carbonate, talc, titanium dioxide, dicalcium phosphate, and the like. Bulking agents may be used in amounts up to about 90%, by weight of the final gum composition, with amounts from about 40% to about 70%, by weight of the gum composition being preferred , with from about 50% to about 65%, by weight, being more preferred and from about 55% to about 60%, by weight of the chewing gum composition, being most preferred.

The sweetening agent used may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sweetening agents such as monosaccharides, disaccharides and polysaccharides such as xylose, ribulose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, glycyrrhizin, and sugar alcohols such as sorbitol, mannitol, maltitol, hydrogenated starch hydrolysates and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and the like;
(c) dipeptide based sweeteners, such as L-aspartic acid derived sweeteners, such as L-aspartyl-L-phenylalanine methyl ester (Aspartame) and materials described in United States patent no. 3,492,131, L-alpha-aspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenylglycine, L-aspartyl-2,5-dihydro-L-phenylalanine; L-aspartyl-L-(1-cyclohexen)-alanine, and the like;
(d) water-soluble sweeteners derived from naturally occurring water-soluble sweeteners, such as chlorinated derivatives of ordinary sugar (sucrose), known, for example, under the product designation of Sucralose; and
(e) protein based sweeteners such as thaumaoccous danielli (Thaumatin I and II).

In general, an effective amount of sweetener is utilized to provide the level of bulk and/or sweetness desired, and this amount will vary with the sweetener selected. This amount of sweetener will normally be present in amounts from about 0.0025% to about 90%, by weight of the gum composition, depending upon the sweetener used. The exact range of amounts for each type of sweetener is well known in the art and is not the subject of the present invention. The amount of sweetener ordinarily necessary to achieve the desired level of sweetness is independent from the flavor level achieved from flavor oils.

Preferred sugar based-sweeteners are sugar (sucrose), corn syrup and mixtures thereof. Preferred sugarless sweeteners are the sugar alcohols, artificial sweeteners, dipeptide based sweeteners and mixtures thereof. Preferably, sugar alcohols are used in the sugarless compositions because these sweeteners can be used in amounts which are sufficient to provide bulk as well as the desired level of sweetness. Preferred sugar alcohols are selected from the group consisting of sorbitol, xylitol, maltitol, mannitol, and mixtures thereof. More preferably, sorbitol or a mixture of sorbitol and mannitol is utilized. The gamma form of sorbitol is preferred. An artificial sweetener or dipeptide based sweetener is preferably added to the gum compositions which contain sugar alcohols.

The coloring agents useful in the gum compositions are used in amounts effective to produce the desired color. These coloring agents include pigments which may be incorporated in amounts up to about 6% by weight of the gum composition. A preferred pigment, titanium dioxide, may be incorporated in amounts up to about 2%, and preferably less than about 1% by weight of the composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No.2, which is the disodium salt of 5,5-indigotindisulfonic acid. Similarly, the dye known as F.D.& C. Green No.1 comprises a triphenylmethane dye and is the monosodium salt of 4-[4-(N-ethyl-p-sulfoniumbenzylamino) diphenylmethylene]-[1-(N-ethyl-N-p-sulfoniumbenzyl)-delta-2,5-cyclohexadienoimine]. A full recitation of all F.D.& C. colorants and their corresponding chemical structures may be found in the Kirk-Othmer Encyclopedia of Chemical Technology, 3rd Edition, in volume 5 at pages 857-884, which text is incorporated herein by reference.

Suitable oils and fats usable in gum compositions include partially hydrogenated vegetable or animal fats, such as coconut oil, palm kernel oil, beef tallow, lard, and the like. These ingredients when used are generally present in amounts up to about 7%, by weight, and preferably up to about 3.5%, by weight of the gum composition.

In accordance with this invention, therapeutically effective amounts of the therapeutic compositions of the present invention may be admixed into a chewing gum. These amounts are readily determined by those skilled in the art without the need for undue experimentation. In a preferred embodiment, the final chewing gum composition will comprise the therapeutic composition in an amount from about 0.1% to about 10% and a chewing gum composition in a quantity sufficient to bring the total amount of composition to 100%, by weight of the chewing gum composition. In a more preferred embodiment, the final chewing gum composition will comprise the therapeutic composition in an amount from about 0.1% to about 5%, and in a most preferred embodiment, the final chewing gum composition will comprise the therapeutic composition in an amount from about 0.1% to about 2%, and a chewing gum composition in a quantity sufficient to bring the total amount of composition to 100%, by weight of the chewing gum composition.

The present invention extends to methods of making the therapeutic chewing gum compositions. The therapeutic compositions may be incorporated into an otherwise conventional chewing gum composition busing standard techniques and equipment known to those skilled in the art. The apparatus useful in accordance with the present invention comprises mixing and heating apparatus well known in the chewing gum manufacturing arts, and therefore the selection of the specific apparatus will be apparent to the artisan.

For example, a gum base is heated to a temperature sufficiently high enough to soften the base without adversely effecting the physical and chemical make up of the base. The optimum temperatures utilized may vary depending upon the composition of the gum base used, but such temperatures are readily determined by those skilled in the art without undue experimentation.

The gum base is conventionally melted at temperatures that range from about 60^{o} C. to about 120^{o} C. for a period of time sufficient to render the base molten. For example, the gum base may be heated under these conditions for a period of about thirty minutes just prior to being admixed incrementally with the remaining ingredients of the base such as the plasticizer, fillers, the bulking agent and/or sweeteners, the softener and coloring agents to plasticize the blend as well as to modulate the hardness, viscoelasticity and formability of the base. The chewing gum base is then blended with the therapeutic composition of the present invention which may have been previously blended with other traditional ingredients. Mixing is continued until a uniform mixture of gum composition is obtained. Thereafter the gum composition mixture may be formed into desirable chewing gum shapes.

In a specific embodiment, the invention is directed at a therapeutic pharmaceutical composition for preventing and reducing injury to mammalian cells, and increasing the resuscitation rate of injured mammalian cells, which comprises:
(A) a therapeutically effective amount of a therapeutic composition selected from the group consisting of:
   (1)
      (a) pyruvate selected from the group consisting of pyruvic acid, pharmaceutically acceptable salts of pyruvic acid, and mixtures thereof;
      (b) an antioxidant; and
      (c) a mixture of saturated and unsaturated fatty acids wherein the fatty acids are those fatty acids required for the resuscitation of injured mammalian cells;
      and
(B) a pharmaceutically acceptable carrier.

The pharmaceutically acceptable carrier may be selected from the group consisting of pharmaceutical appliances, topical vehicles, and ingestible vehicle.

In another specific embodiment, the invention is directed at a method for preparing a therapeutic pharmaceutical composition for preventing and reducing injury to mammalian cells, and increasing the resuscitation rate of injured mammalian cells, which comprises the steps of :
(A) providing a therapeutically effective amount of a therapeutic composition selected from the group consisting of:
   (1)
      (a) pyruvate selected from the group consisting of pyruvic acid, pharmaceutically acceptable salts of pyruvic acid, and mixtures thereof;
      (b) an antioxidant; and
      (c) a mixture of saturated and unsaturated fatty acids wherein the fatty acids are those fatty acids required for the resuscitation of injured mammalian cells;
      and
(B) providing a pharmaceutically acceptable carrier; and
(C) admixing the therapeutic composition from step (A) and the pharmaceutically acceptable carrier from step (B) to form a therapeutic pharmaceutical composition.

The present invention is further illustrated by the following examples which are not intended to limit the effective scope of the claims. All parts and percentages in the examples and throughout the specification and claims are by weight of the final composition unless otherwise specified.

### EXAMPLES 1-26

These examples demonstrate a comparison of the viability of U937 monocytic cells after exposure of the cells to various antioxidants and combinations of antioxidants. These examples also demonstrate a comparison of the levels of hydrogen peroxide produced by U937 monocytic cells and mammalian epidermal keratinocytes after exposure of the cells to various antioxidants and combinations of antioxidants.

Mammalian epidermal keratinocytes and monocytes were employed to examine the ability of various antioxidants to reduce levels of hydrogen peroxide in these cells. Hydrogen peroxide was measured after the cells were exposed to ultraviolet light in the wavelength range from 290 to 320 nm (UV-B) or to the inflammatory compound 12-0-tetradecanoyl-phorbol-13-acetate (TPA). Sodium pyruvate was tested at various concentrations to determine the effect of concentrations of this antioxidant on the hydrogen peroxide production by epidermal cells and monocytes. Magnesium pyruvate, calcium pyruvate, zinc pyruvate, and combinations of sodium pyruvate with ascorbic acid, lactic acid, and Vitamin E were then tested to determine the effect of these salts and combinations of antioxidants on the hydrogen peroxide production by epidermal cells and monocytes.

Mammalian epidermal keratinocytes were isolated by trypsinization of epithelial sheets and grown in modified basal MCDB 153 medium supplemented with epidermal growth factor, bovine pituitary extract, and hydrocortisone. Cells were maintained in a humidified incubator with 5% carbon dioxide at 37 ^{o}C. Keratinocytes were seeded in 60 mm culture dishes at a cell density of 3 x 10⁵ cells per dish and the cultures were exposed to 1 M.E.D. dose of ultraviolet-B light (100 mJ/cm²) or treated with 100 ng/ml of TPA.

U937 monocytic cells are a cultured cell line grown in RPMI media with 10% fetal calf serum. Cells were maintained in a 60 mm culture dish at 5% carbon dioxide at 37 ^{o}C. at a seeding density not exceeding 1 x 10⁶ cells per dish.

Sodium pyruvate, lactic acid, ascorbic acid, and Vitamin E were dissolved in distilled water. The concentrations of the sodium pyruvate solutions prepared were 1 mM, 10 mM, 50 mM, 100 mM, and 200 mM. The concentrations of the lactic acid solutions prepared were 1.0%, 0.1%, and 0.05%. The concentrations of the ascorbic acid solutions prepared were 1.0%, 0.1%, 0.05%, and 0.025%. The concentrations of the Vitamin E solutions prepared were 1 U, 10 U, 50 U, and 100 U. The test solutions were adjusted to a pH value of 7.4 with 1.0N sodium hydroxide solution and then sterile filtered. The appropriate concentration of test solution or combination of test solutions was added to the cells immediately prior to exposure of the cells to ultraviolet light-B or TPA [100ng/ml]. Stock solutions were prepared so that the vehicle did not constitute more than 1% of the total volume of the culture media.

Intracellular hydrogen peroxide production by mammalian epidermal keratinocytes and U937 monocytes was measured using dichlorofluorescein diacetate (DCFH-DA, Molecular Probes, Eugene, Ore.). DCFH-DA is a non-polar non-fluorescent compound that readily diffuses into cells where it is hydrolyzed to the polar non-fluorescent derivative DCFH which then becomes trapped within the cells. In the presence of intracellular hydrogen peroxide, DCFH is oxidized to the highly fluorescent compound DCF. Hence, cellular fluorescence intensity is directly proportional to the level of intracellular hydrogen peroxide produced. Cellular fluorescence intensity can be monitored by fluorimetry and by flow cytometry.

Mammalian epidermal keratinocytes and U937 cultured monocytes (1 x 10⁶ per dish) were incubated at 37 ^{o}C. with 5 uM of DCFH-DA. Production of hydrogen peroxide was measured using a Coulter Profile analytical flow cytometer. Linear and log intensity of green fluorescence data was collected. For each analysis, a quantity of 10,000 to 20,000 events was accumulated. Optical alignment for the instrument was performed daily. Coefficients of variation for forward angle light scatter and integrated green fluorescence were generally less than two. Each analysis was repeated three times and the quantitation of fluorescence was expressed in terms of femtomoles (fmol, 10⁻¹⁵ moles) of DCF oxidized per cell, which is a direct measure of the intracellular hydrogen peroxide produced. Alternatively, in the saturated and unsaturated fatty acid examples in examples 27-52, fluorimetry was used to assess the DCF oxidation per cell.

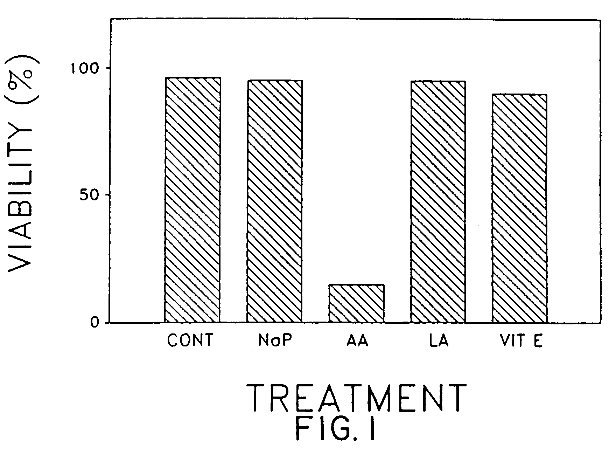

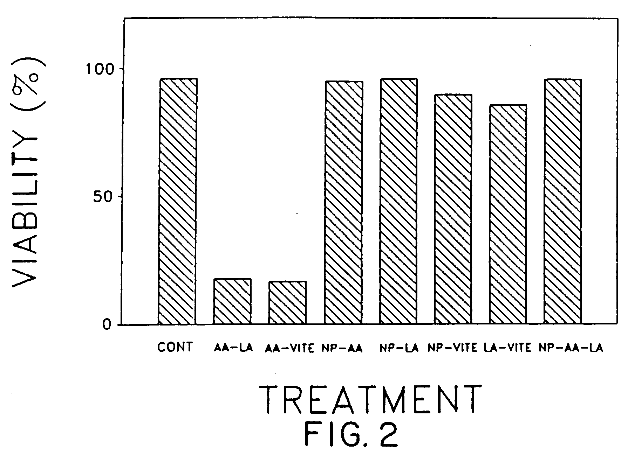

The viability of the U937 monocytic cells after exposure of the cells to various antioxidants for 24 hours was measured. The viability of the cells was determined by exposing the cells to the dye propidium iodide. Permeable cell membranes which absorbed the dye were not considered viable. The viability of the cells was represented as the percentage of cells that excluded propidium iodide. FIGURE 1 depicts in bar graph format the viability of U937 monocytic cells after exposure of the cells to no antioxidant (Example 1, control), to sodium pyruvate (Example 2), to ascorbic acid (Example 3), to lactic acid (Example 4), and to Vitamin E (Example 5). FIGURE 2 depicts in bar graph format the viability of U937 monocytic cells after exposure of the cells to various combinations of antioxidants. Specifically, the viability of U937 monocytic cells was measured after exposure to no antioxidant (Example 6, control), to ascorbic acid and lactic acid (Example 7), to ascorbic acid and Vitamin E (Example 8), to sodium pyruvate and ascorbic acid (Example 9), to sodium pyruvate and lactic acid (Example 10), to sodium pyruvate and Vitamin E (Example 11), to lactic acid and Vitamin E (Example 12), and to sodium pyruvate, ascorbic acid, and lactic acid (Example 13).

FIGURE 1 shows that ascorbic acid is cytotoxic to monocytes at concentrations as low as 0.25%. FIGURE 2 shows that the cytotoxicity of ascorbic acid was reversed by the addition off 10 mM of sodium pyruvate. FIGURES 1 and 2 show that the viability rate of 15% to 20% of the cells when treated with ascorbic acid was increased to 95% to 98% upon addition off sodium pyruvate. Lactic acid and Vitamin E did not reverse the cytotoxicity of ascorbic acid.

Sodium pyruvate was then tested at various concentrations to determine the effect of concentrations of this antioxidant on the hydrogen peroxide production by epidermal cells and monocytes. Mammalian epidermal keratinocytes and monocytes were exposed to (a) 1 M.E.D. dose of ultraviolet light-B and (b) 100 ng/ml of 12-O-tetradecanoylphorbol-13-acetate (TPA) in the presence of sodium pyruvate at the following concentrations: 200 mM, 100 mM, 50 mM, 10 mM, 1 mM.

The optimum concentration of sodium pyruvate to reduce the hydrogen peroxide production by epidermal cells and monocytes was found to be 10 mM. Concentrations of sodium pyruvate of 50 mM and above were cytotoxic to both epidermal keratinocytes and monocytes.

Magnesium pyruvate, calcium pyruvate, zinc pyruvate, ascorbic acid, lactic acid, and Vitamin E, and combinations of sodium pyruvate with ascorbic acid, lactic acid, and Vitamin E were then tested to determine the effect of these salts and combinations of antioxidants on the hydrogen peroxide production by epidermal cells and monocytes. The following test solutions were prepared.
(a) sodium pyruvate [10 mM];
(b) zinc salt [10 mM];
(c) magnesium salt [10 mM];
(d) calcium salt [10 mM];
(e) sodium pyruvate [10 mM] and ascorbic acid [0.025%];
(f) sodium pyruvate [10 mM] and lactic acid [0.05%];
(g) sodium pyruvate [10 mM), lactic acid, [0.05%], and ascorbic acid [0.025%];
(h) lactic acid [1.0%, 0.1%, and 0.05%];
(i) ascorbic acid [1.0%, 0.1%, 0.05%, and 0.025%];
(j) Vitamin E [1 U, 10 U, 50 U, and 100 U]; and
(k) vehicle solvent controls.

There was no significant difference among the zinc, magnesium, and calcium salts of pyruvic acid on the hydrogen peroxide production by epidermal cells and monocytes. The zinc and calcium salts of pyruvic acid induced differentiation of keratinocytes. For convenience, the sodium salt was used in subsequent tests.

The optimum concentration of lactic acid to reduce the hydrogen peroxide production by epidermal cells and monocytes was found to be 0.05%. The optimum concentration of ascorbic acid was found to be 0.025%. The higher concentrations of both of these compounds were found to be cytotoxic to both types of cells. The optimum concentration of Vitamin E was found to be 50 U.

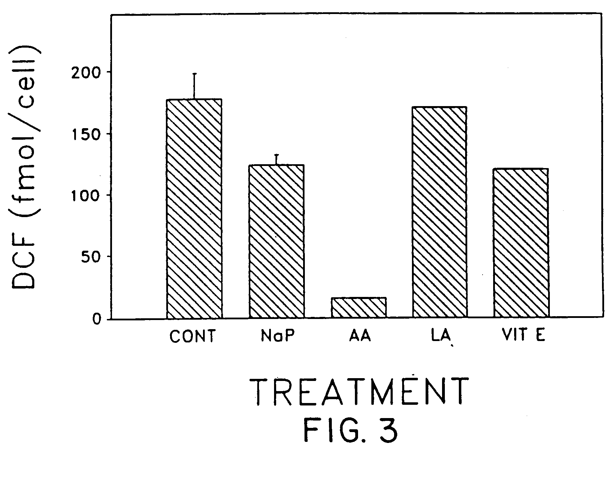

FIGURE 3 depicts in bar graph format the levels of hydrogen peroxide produced by U937 monocytic cells after exposure of the cells to no antioxidant (Example 14, control), to sodium pyruvate (Example 15), to ascorbic acid (Example 16), to lactic acid (Example 17), and to Vitamin E (Example 18). Sodium pyruvate and Vitamin E significantly reduced the hydrogen peroxide production by monocytes.

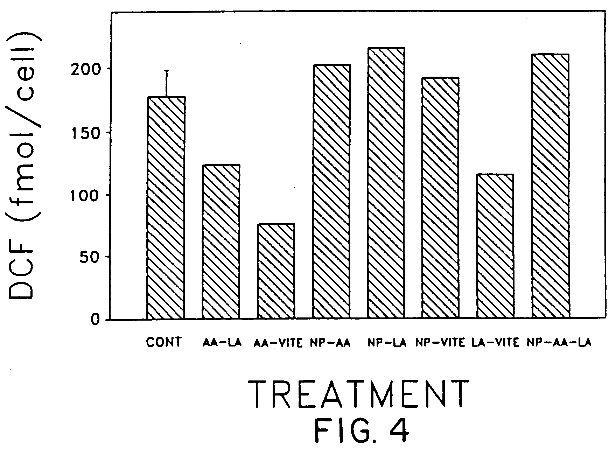

FIGURE 4 depicts in bar graph format the levels of hydrogen peroxide produced by U937 monocytic cells after exposure of the cells to various combinations of antioxidants. Specifically, the levels of hydrogen peroxide produced by U937 monocytic cells were measured after exposure to no antioxidant (Example 19, control), to ascorbic acid and lactic acid (Example 20), to ascorbic acid and Vitamin E (Example 21), to sodium pyruvate and ascorbic acid (Example 22), to sodium pyruvate and lactic acid (Example 23), to sodium pyruvate and Vitamin E (Example 24), to lactic acid and Vitamin E (Example 25), and to sodium pyruvate, ascorbic acid, and lactic acid (Example 26). The combination of lactic acid (0.05%) and Vitamin E (50 U) significantly reduced the hydrogen peroxide production by monocytes.

The morphological alterations in epidermal keratinocytes were observed in control cultures and in cultures exposed to ultraviolet-B. Cells in the layer closest to the dermis are basal keratinocytes. These cells proliferate and migrate into the spinous and granular layers of the epidermis where the cells begin to differentiate. The differentiation pattern results in cells enucleating and forming cornified envelopes at the uppermost portion of the epidermis, the statum corneum. The differentiation of keratinocytes is controlled by the levels of calcium, magnesium, and other elements in the medium. Cells in culture systems promoting differentiation appear as an epidermal sheet forming attachments or tight junctions with each other. Keratinocytes that become nonadherent or float in the media were considered responding to a cytotoxic event.

The following morphological alterations in the mammalian epidermal keratinocytes were observed for the following control cultures:
10 mM Sodium Pyruvate: Tight junctions of cells were formed and the proliferation rate of the cells was higher than the rate of the control cells.
0.025% Ascorbic Acid: Cells were floating in a cytotoxic response to ascorbic acid.
0.025% Ascorbic acid and 10 mM Sodium Pyruvate: Few tight junctions of cells were observed and cells appeared similar to the cells in the sodium pyruvate culture.
0.05% Lactic Acid: Cells appeared dramatically altered as an epidermal sheet and as flat granular cells.
0.05% Lactic Acid and 10 mM Sodium Pyruvate: Cells formed an epidermal sheet but appeared smaller than the cell in the lactic acid culture.
50 U Vitamin E: Cells appeared the same as the cells in the control culture.

### EXAMPLES 27-52

These examples demonstrate a comparison of the levels of hydrogen peroxide produced by U937 monocytic cells and epidermal keratinocytes after exposure of the cells to various combinations of antioxidants with and without a mixture of saturated and unsaturated fatty acids.

Mammalian epidermal keratinocytes and U937 monocytic cells and the test solutions of sodium pyruvate, lactic acid, ascorbic acid, and Vitamin E were prepared as describe above for Examples 1-26. Intracellular hydrogen peroxide production by the mammalian epidermal keratinocytes and U937 monocytes was also measured as described above.

A mixture of fatty acids derived from chicken fat was prepared for addition to the cultured cells by mixing 0.1% of the chicken fat with the culture media. At the temperature of the culture media, 37 ^{o}C., the chicken fat was miscible. This chicken fat mixture was added to cultures of cells prior to exposure of the cells to ultraviolet-B light or TPA treatment.

As set out in examples 1-26, mammalian epidermal keratinocytes and monocytes were exposed to (a) 1 M.E.D. dose of ultraviolet light-B and (b) 100 ng/ml of 12-O-tetradecanoylphorbol-13-acetate in the presence of various antioxidants and combinations of antioxidants with and without a mixture of saturated and unsaturated fatty acids [0.1%, 0.5%, and 1.0% chicken fat].

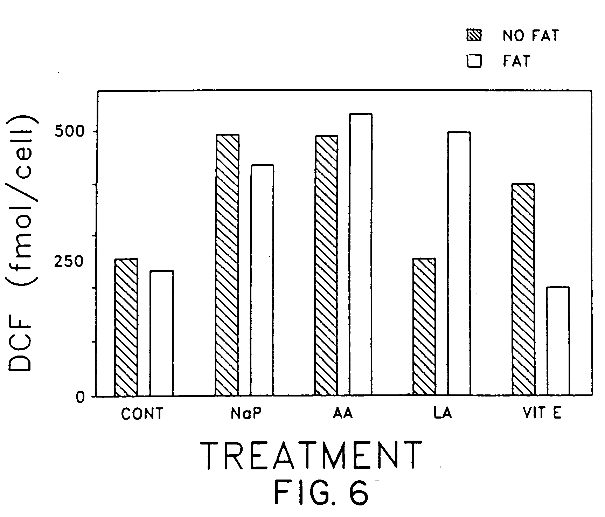

FIGURE 6 depicts in bar graph format the levels of hydrogen peroxide produced by epidermal keratinocytes after exposure of the cells to various antioxidants with and without a mixture of saturated and unsaturated fatty acids. Specifically, the levels of hydrogen peroxide produced by epidermal keratinocytes were measured after exposure to no antioxidant without fatty acids (Example 33, control) and with fatty acids (Example 34), to sodium pyruvate without fatty acids (Example 35) and with fatty acids (Example 36), to ascorbic acid without fatty acids (Example 37) and with fatty acids (Example 38), to lactic acid without fatty acids (Example 39) and with fatty acids (Example 40), and to Vitamin E without fatty acids (Example 41) and with fatty acids (Example 42). The ability of sodium pyruvate and Vitamin E to reduce the hydrogen peroxide production by epidermal keratinocytes was increased in the presence of fatty acids. The most effective combinations to reduce the hydrogen peroxide production of epidermal keratinocytes were sodium pyruvate in combination with a mixture saturated and unsaturated fatty acids and Vitamin E in combination with a mixture of saturated and unsaturated fatty acids.

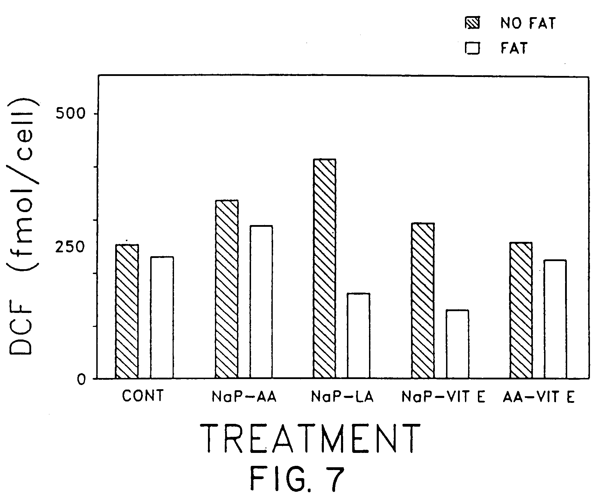

FIGURE 7 depicts in bar graph format the levels of hydrogen peroxide produced by epidermal keratinocytes after exposure of the cells to various combinations of antioxidants with and without a mixture of saturated and unsaturated fatty acids. Specifically, the levels of hydrogen peroxide produced by epidermal keratinocytes were measured after exposure to no antioxidant without fatty acids (Example 43, control) and with fatty acids (Example 44), to sodium pyruvate and ascorbic acid without fatty acids (Example 45) and with fatty acids (Example 46), to sodium pyruvate and lactic acid without fatty acids (Example 47) and with fatty acids (Example 48), to sodium pyruvate and Vitamin E without fatty acids (Example 49) and with fatty acids (Example 50), and to ascorbic acid and Vitamin E without fatty acids (Example 51) and with fatty acids (Example 52). The ability of all combinations of antioxidants to reduce the hydrogen peroxide production by epidermal keratinocytes was increased in the presence of fatty acids. In order of potency, the most effective combinations to reduce the hydrogen peroxide production of epidermal keratinocytes were sodium pyruvate and Vitamin E, sodium pyruvate and lactic acid, and Vitamin E, each in combination with a mixture of saturated and unsaturated fatty acids (0.5%).

## Claims

1. A therapeutic composition for preventing and reducing injury to mammalian cells, and increasing the resuscitation rate of injured mammalian cells, which comprises
(a) a pyruvate selected from the group consisting of pyruvic acid, pharmaceutically acceptable salts of pyruvic acid, and mixtures thereof;
(b) an antioxidant; and
(c) a mixture of saturated and unsaturated fatty acids.

2. The composition according to claim 1, wherein the mammalian cells comprise epidermal keratinocytes or monocytes.

3. The composition according to claims 1 or 2, wherein the pyruvate is selected from the group consisting of pyruvic acid, lithium pyruvate, sodium pyruvate, potassium pyruvate, magnesium pyruvate, calcium pyruvate, zinc pyruvate, manganese pyruvate, and mixtures thereof.

4. The composition according to claim 3, wherein the pyruvate is sodium pyruvate.

5. The composition according to claims 1 to 4, wherein the antioxidant is selected from the group consisting of retinol, 3,4-didehydroretinol, alpha-carotene, beta-carotene, gamma-carotene, delta-carotene, ascorbic acid, alpha-tocopherol, beta-tocopherol, gamma-tocopherol, delta-tocopherol, and mixtures thereof.

6. The composition according to claim 5, wherein the antioxidant is alpha-tocopherol.

7. The composition according to claim 1 to 6, wherein the mixture of saturated and unsaturated fatty acids comprises lauric acid, myristic acid, myristoleic acid, pentadecanoic acid, palmitic acid, palmitoleic acid, margaric acid, margaroleic acid, stearic acid, oleic acid, linoleic acid, linolenic acid, arachidic acid, and gaddoleic acid.

8. The composition according to claim 7, wherein the mixture of saturated and unsaturated fatty acids comprises human fat, chicken fat, cow fat, sheep fat, horse fat, pig fat, and whale fat.

9. The composition according to claims 1 to 8 wherein the single components pyruvate, antioxidant and fatty acids are present in the therapeutic composition in an amount from about 10% to about 50%, by weight of the therapeutic composition.

10. A process for preparing a therapeutic composition for preventing and reducing injury to mammalian cells, and increasing the resuscitation rate of injured mammalian cells, which comprises the steps of admixing the ingredients according to anyone of the preceding claims 1 to 9.

11. An augmented pharmaceutical composition having an enhanced ability to prevent and reduce injury to mammalian cells which comprises:
(A) A therapeutic composition according to anyone of claims 1 to 9
(B) a medicament useful for treating injured mammalian cells.

12. The augmented pharmaceutical composition according to claim 11, wherein the medicament is selected from anti-inflammatories, antibacterials, antiseptics, burn relief medications, sun burn medications, acne preparations, insect bite and sting medications, wound cleansers and wound dressings, stroke medications, autoimmune disease medications, arthritis medications, ulcer medications, and cancer medications.

13. A cytoprotective pharmaceutical composition for preventing and reducing injury to mammalian cells from a medicament having cytotoxic properties which comprises:
(A) a therapeutic composition according to anyone of claims 1 to 9 and
(B) a medicament having cytotoxic properties.

14. The cytoprotective pharmaceutical composition according to claim 13, wherein the medicament having cytotoxic properties is selected from the group consisting of epithelial cell cohesiveness reducers, dermatological abradants, anti-inflammatories, lipid regulating agents, centrally acting anticholinesterases, chemotherapeutic drugs, and gastric irritants.

15. The cytoprotective pharmaceutical composition according to claim 14, wherein the medicament having cytotoxic properties is selected from the group consisting of gemfibrozil, lovastatin, tacrine, and doxorubicin.

16. A wound healing pharmaceutical composition to increase the resuscitation rate of injured mammalian cells which comprises:
(A) a first wound healing component comprising a mixture of saturated and unsaturated fatty acids and
(B) a second wound healing component consisting of:
(a) pyruvate selected from the group consisting of pyruvic acid, pharmaceutically acceptable salts of pyruvic acid, and mixtures thereof; and
(b) an antioxidant.

17. A process for preparing an augmented pharmaceutical composition having an enhanced ability to prevent and reduce injury to mammalian cells which comprises the steps of:
(A) providing a therapeutic composition selected from the group of consisting of:
(a) pyruvate selected from the group consisting of pyruvic acid, pharmaceutically acceptable salts of pyruvic acid, and mixtures thereof;
(b) an antioxidant; and
(c) a mixture of saturated and unsaturated fatty acids.
(B) providing a medicament useful for treating injured mammalian cells; and
(C) admixing the therapeutic composition from step (A) with the medicament from step (B) to prepare the augmented pharmaceutical composition.

18. A process for preparing a cytoprotective pharmaceutical composition for preventing and reducing injury to mammalian cells from a medicament having cytotoxic properties which comprises the steps of:
(A) providing a therapeutic composition selected from the group consisting of:
(a) pyruvate selected from the group consisting of pyruvic acid, pharmaceutically acceptable salts of pyruvic acid, and mixtures thereof;
(b) an antioxidant; and
(c) a mixture of saturated and unsaturated fatty acids;
(B) providing a medicament having cytotoxic properties; and
(C) admixing the therapeutic composition from step (A) with the medicament from step (B) to prepare the cytoprotective pharmaceutical composition.

19. A process for preparing a wound healing pharmaceutical composition to increase the resuscitation rate of injured mammalian cells which comprises the steps of:
(A) providing a first wound healing component comprising a mixture of saturated and unsaturated fatty acids; and
(B) providing a second wound healing component consisting of:
(a) pyruvate selected from the group consisting of pyruvic acid, pharmaceutically acceptable salts of pyruvic acid, and mixtures thereof and
(b) an antioxidant; and
(C) admixing the first wound healing component from step
(A) with the second wound healing component from step
(B) to prepare the wound healing pharmaceutical composition.

20. A process for preserving mammalian cells in a culture medium which comprises the steps of:
(A) providing a therapeutic composition selected from the group of consisting of:
(a) pyruvate selected from the group consisting of pyruvic acid, pharmaceutically acceptable salts of pyruvic acid, and mixtures thereof;
(b) an antioxidant; and
(c) a mixture of saturated and unsaturated fatty acids wherein the fatty acids are those fatty acids required for the resuscitation of injured mammalian cells;
(B) providing mammalian cells in a culture medium; and
(C) contacting the therapeutic composition from step (A) with the mammalian cells in the culture medium from step (B).

21. A therapeutic pharmaceutical composition for preventing and reducing injury to mammalian cells and increasing the resuscitation rate of injured mammalian cells, which comprises:
(A) a therapeutically effective amount of a therapeutic composition according to anyone of claims 1 to 9 and
(B) a pharmaceutically acceptable carrier.

22. The pharmaceutical composition according to claim 21, wherein the pharmaceutically acceptable carrier is a pharmaceutical appliance.

23. The pharmaceutical composition according to claim 21, wherein the pharmaceutically acceptable carrier is a topical vehicle.

24. The pharmaceutical composition according to claim 21, wherein the pharmaceutically acceptable carrier is an ingestible vehicle.

25. A process for preparing a therapeutic pharmaceutical composition for preventing and reducing injury to mammalian cells, and increasing the resuscitation rate of injured mammalian cells, which comprises the steps of:
(A) providing a therapeutically effective amount of a therapeutic composition according to anyone of claims 1 to 9,
(B) providing a pharmaceutically acceptable carrier; and
(C) admixing the therapeutic composition from step (A) and the pharmaceutically acceptable carrier from step (B) to form a therapeutic pharmaceutical composition.

26. A process according to claim 25 for preparing a therapeutic pharmaceutical composition for preventing and reducing injury to mammalian cells, and increasing the resuscitation rate of injured mammalian cells, which comprises the steps of:
(A) providing a therapeutically effective amount of a therapeutic composition selected from the group consisting of:
(a) pyruvate selected from the group consisting of pyruvic acid, pharmaceutically acceptable salts of pyruvic acid, and mixtures thereof;
(b) an antioxidant; and
(c) a mixture of saturated and unsaturated fatty acids wherein the fatty acids are those fatty acids required for the resuscitation of injured mammalian cells;
(B) providing a pharmaceutically acceptable carrier; and
(C) admixing the therapeutic composition from step (A) and the pharmaceutically acceptable carrier from step (B) to form a therapeutic pharmaceutical composition.

## Patentansprüche

1. Therapeutisches Mittel zur Verhinderung und Verminderung einer Schädigung bzw. Verletzung von Säugetierzellen und zur Steigerung der Wiederbelebungsgeschwindigkeit bzw. der Wiederbelebungsrate verletzter Säugetierzellen, umfassend
(a) ein Pyruvat, ausgewählt aus der Gruppe Brenztraubensäure, pharmazeutisch akzeptable Salze der Brenztraubensäure und Mischungen hiervon;
(b) ein Antioxidationsmittel und
(c) ein Gemisch gesättigter und ungesättigter Fettsäuren.

2. Mittel nach Anspruch 1, wobei es sich bei den Säugetierzellen um epidermale Keratinozyten oder Monozyten handelt.

3. Mittel nach Ansprüchen 1 oder 2, wobei das Pyruvat aus der Gruppe Brenztraubensäure, Lithiumpyruvat, Natriumpyruvat, Kaliumpyruvat, Magnesiumpyruvat, Calciumpyruvat, Zinkpyruvat, Manganpyruvat und Mischungen hiervon ausgewählt ist.

4. Mittel nach Anspruch 3, wobei das Pyruvat aus Natriumpyruvat besteht.

5. Mittel nach Ansprüchen 1 bis 4, wobei das Antioxidationsmittel aus der Gruppe Retinol, 3,4-Didehydroretinol, alpha-Carotin, beta-Carotin, gamma-Carotin, delta-Carotin, Ascorbinsäure, alpha-Tocopherol, beta-Tocopherol, gamma-Tocopherol, delta-Tocopherol und Mischungen hiervon ausgewählt ist.

6. Mittel nach Anspruch 5, wobei das Antioxidationsmittel aus alpha-Tocopherol besteht.

7. Mittel nach Ansprüchen 1 bis 6, wobei das Gemisch aus gesättigten und ungesättigten Fettsäuren Laurinsäure, Myristinsäure, Myristoleinsäure, Pentadecansäure, Palmitinsäure, Palmitoleinsäure, Margarinsäure, Margaroleinsäure, Stearinsäure, Ölsäure, Linolsäure, Linolensäure, Arachidonsäure und Gadoleinsäure umfaßt.

8. Mittel nach Anspruch 7, wobei das Gemisch aus gesättigten und ungesättigten Fettsäuren menschliches Fett, Hühnerfett, Rinderfett, Schaffett, Pferdefett, Schweinefett und Walfett umfaßt.

9. Mittel nach Ansprüchen 1 bis 8, wobei die einzelnen Komponenten Pyruvat, Antioxidationsmittel und Fettsäuren in dem therapeutischen Mittel in einer Menge von etwa 10 bis etwa 50 Gew.-% des therapeutischen Mittels enthalten sind.

10. Verfahren zur Gewinnung eines therapeutischen Mittels zur Verhinderung und Verminderung einer Schädigung bzw. Verletzung von Säugetierzellen und zur Steigerung der Wiederbelebungsgeschwindigkeit bzw. -rate verletzter Säugetierzellen durch Vermischen der Bestandteile gemäß einem der vorhergehenden Ansprüche 1 bis 9.

11. Verstärkte Arzneimittelzubereitung mit gesteigerter Fähigkeit zur Verhinderung und Verminderung einer Schädigung bzw. Verletzung von Säugetierzellen, umfassend:
(A) Ein therapeutisches Mittel nach einem der Ansprüche 1 bis 9 und
(B) ein zur Behandlung geschädigter bzw. verletzter Säugetierzellen brauchbares Medikament.

12. Verstärkte Arzneimittelzubereitung nach Anspruch 11, wobei das Medikament aus entzündungshemmenden Mitteln, antibakteriellen Mitteln, Antiseptika, Verbrennungen lindernden Medikamenten, Mitteln gegen Sonnenbrand, Aknepräparaten, Mitteln gegen Insektenbisse und -stiche, Wundreinigungsmitteln und Wundverbänden, Mitteln gegen Schlaganfall, Mitteln gegen Autoimmunerkrankungen, Mitteln gegen Arthritis, Mitteln gegen Geschwüre und Krebsmitteln ausgewählt ist.

13. Zellenschützende Arzneimittelzubereitung zur Verhinderung und Verminderung einer Schädigung bzw. Verletzung von Säugetierzellen durch ein Medikament mit cytotoxischen Eigenschaften, umfassend
(A) Ein therapeutisches Mittel nach einem der Ansprüche 1 bis 9 und
(B) ein Medikament mit cytotoxischen Eigenschaften.

14. Zellenschützende Arzneimittelzubereitung nach Anspruch 13, wobei das Medikament mit cytotoxischen Eigenschaften aus der Gruppe Mittel zur Verminderung der Kohäsivkraft von Epithelzellen, dermatologische Schleifmittel bzw. Ablösung erzeugende Mittel, entzündungshemmende Mittel, Lipidsteuermittel, zentral wirkende Anticholinesterasen, chemotherapeutische Mittel und Magenreizmittel ausgewählt ist.

15. Zellenschützende Arzneimittelzubereitung nach Anspruch 14, wobei das Medikament mit cytotoxischen Eigenschaften aus der Gruppe Gemfibrozil, Lovastatin, Tacrin und Doxorubicin ausgewählt ist.

16. Arzneimittelzubereitung für die Wundheilung zur Steigerung der Wiederbelebungsgeschwindigkeit bzw. -rate geschädigter bzw. verletzter Säugetierzellen, umfassend
(A) eine erste Wundheilungskomponente, die ein Gemisch aus gesättigten und ungesättigten Fettsäuren umfaßt, und
(B) eine zweite Wundheilungskomponente, bestehend aus
(a) einem Pyruvat, ausgewählt aus der Gruppe Brenztraubensäure, pharmazeutisch akzeptable Salze der Brenztraubensäure und Mischungen hiervon, und
(b) einem Antioxidationsmittel.

17. Verfahren zur Gewinnung einer verstärkten Arzneimittelzubereitung mit gesteigerter Fähigkeit zur Verhinderung und Verminderung einer Schädigung bzw. Verletzung von Säugetierzellen in folgenden Stufen:
(A) Bereitstellen eines therapeutischen Mittels, ausgewählt aus der aus
(a) einem Pyruvat, ausgewählt aus der Gruppe Brenztraubensäure, pharmazeutisch akzeptable Salze der Brenztraubensäure und Mischungen hiervon;
(b) einem Antioxidationsmittel und
(c) einem Gemisch aus gesättigten und ungesättigten Fettsäuren
bestehenden Gruppe;
(B) Bereitstellen eines Medikaments mit der Eignung zur Behandlung geschädigter bzw. verletzter Säugetierzellen und
(C) Vermischen des therapeutischen Mittels aus Stufe (A) mit dem Medikament aus Stufe (B) zur Gewinnung der verstärkten Arzneimittelzubereitung.

18. Verfahren zur Gewinnung einer zellenschützenden Arzneimittelzubereitung zur Verhinderung und Verminderung einer Schädigung bzw. Verletzung von Säugetierzellen durch ein Medikament mit cytotoxischen Eigenschaften in folgenden Stufen:
(A) Bereitstellen eines therapeutischen Mittels, ausgewählt aus der aus
(a) einem Pyruvat, ausgewählt aus der Gruppe Brenztraubensäure, pharmazeutisch akzeptable Salze der Brenztraubensäure und Mischungen hiervon;
(b) einem Antioxidationsmittel und
(c) einem Gemisch aus gesättigten und ungesättigten Fettsäuren
bestehenden Gruppe;
(B) Bereitstellen eines Medikaments mit cytotoxischen Eigenschaften und
(C) Vermischen des therapeutischen Mittels aus Stufe (A) mit dem Medikament aus Stufe (B) zur Gewinnung der zellenschützenden Arzneimittelzubereitung.

19. Verfahren zur Gewinnung einer Arzneimittelzubereitung für die Wundheilung zur Steigerung der Wiederbelebungsgeschwindigkeit bzw. -rate geschädigter bzw. verletzter Säugetierzellen in folgenden Stufen:
(A) Bereitstellen einer ersten Wundheilungskomponente, die ein Gemisch aus gesättigten und ungesättigten Fettsäuren umfaßt;
(B) Bereitstellen einer zweiten Wundheilungskomponente, bestehend aus:
(a) einem Pyruvat, ausgewählt aus der Gruppe Brenztraubensäure, pharmazeutisch akzeptable Salze der Brenztraubensäure und Mischungen hiervon, und
(b) einem Antioxidationsmittel, und
(C) Vermischen der ersten Wundheilungskomponente aus Stufe (A) mit der zweiten Wundheilungskomponente aus Stufe (B) zur Gewinnung der Arzneimittelzubereitung für die Wundheilung.

20. Verfahren zur Konservierung von Säugetierzellen in einem Kulturmedium in folgenden Stufen:
(A) Bereitstellen eines therapeutischen Mittels, ausgewählt aus der aus
(a) einem Pyruvat, ausgewählt aus der Gruppe Brenztraubensäure, pharmazeutisch akzeptable Salze der Brenztraubensäure und Mischungen hiervon;
(b) einem Antioxidationsmittel und
(c) einem Gemisch gesättigter und ungesättigter Fettsäuren, wobei es sich bei den Fettsäuren um die für die Wiederbelebung geschädigter bzw. verletzter Säugetierzellen erforderlichen Fettsäuren handelt;
bestehenden Gruppe;
(B) Bereitstellen von Säugetierzellen in einem Kulturmedium, und
(C) Kontaktieren des therapeutischen Mittels aus Stufe (A) mit den Säugetierzellen in dem Kulturmedium aus Stufe (B).

21. Therapeutische Arzneimittelzubereitung zur Verhinderung und Verminderung einer Schädigung bzw. Verletzung von Säugetierzellen und zur Steigerung der Wiederbelebungsgeschwindigkeit bzw. -rate geschädigter bzw. verletzter Säugetierzellen, umfassend
(A) eine therapeutisch wirksame Menge eines therapeutischen Mittels nach einem der Ansprüche 1 bis 9 und
(B) einen pharmazeutisch akzeptablen Träger.

22. Arzneimittelzubereitung nach Anspruch 21, wobei der pharmazeutisch akzeptable Träger aus einem pharmazeutischen Gerät besteht.

23. Arzneimittelzubereitung nach Anspruch 21, wobei der pharmazeutisch akzeptable Träger aus einem topischen Träger besteht.

24. Arzneimittelzubereitung nach Anspruch 21, wobei der pharmazeutisch akzeptable Träger aus einem einnehmbaren Träger besteht.

25. Verfahren zur Gewinnung einer therapeutischen Arzneimittelzubereitung zur Verhinderung und Verminderung einer Schädigung bzw. Verletzung von Säugetierzellen und zur Steigerung der Wiederbelebungsgeschwindigkeit bzw. -rate von geschädigten bzw. verletzten Säugetierzellen in folgenden Stufen:
(A) Bereitstellen einer therapeutisch wirksamen Menge eines therapeutischen Mittels nach einem der Ansprüche 1 bis 9;
(B) Bereitstellen eines pharmazeutisch akzeptablen Trägers und
(C) Vermischen des therapeutischen Mittels aus Stufe (A) mit dem pharmazeutisch akzeptablen Träger aus Stufe (B) zur Bildung einer therapeutischen Arzneimittelzubereitung.

26. Verfahren nach Anspruch 25 zur Gewinnung einer therapeutischen Arzneimittelzubereitung zur Verhinderung und Verminderung einer Schädigung bzw. Verletzung von Säugetierzellen und zur Steigerung der Wiederbelebungsgeschwindigkeit bzw. -rate von geschädigten bzw. verletzten Säugetierzellen in folgenden Stufen:
(A) Bereitstellen einer therapeutisch wirksamen Menge eines therapeutischen Mittels, ausgewählt aus der aus:
(a) einem Pyruvat, ausgewählt aus der Gruppe Brenztraubensäure, pharmazeutisch akzeptable Salze der Brenztraubensäure und Mischungen hiervon;
(b) einem Antioxidationsmittel und
(c) einem Gemisch gesättigter und ungesättigter Fettsäuren, wobei es sich bei den Fettsäuren um zur Wiederbelebung von geschädigten bzw. verletzten Säugetierzellen erforderliche Fettsäuren handelt;
bestehenden Gruppe;
(B) Bereitstellen eines pharmazeutisch akzeptablen Trägers und
(C) Vermischen des therapeutischen Mittels aus Stufe (A) mit dem pharmazeutisch akzeptablen Träger aus Stufe (B) zur Bildung einer therapeutischen Arzneimittelzubereitung.

## Revendications

1. Une composition thérapeutique pour la prévention et la l'atténuation des lésions des cellules de mammifères et l'augmentation de la vitesse en régénération des cellules de mammifères lésées qui comprend :
(a) un pyruvate sélectionné dans le groupe consistant en acide pyruvique, les sels pharmaceutiquement acceptables de l'acide pyruvique et les mélanges en dérivant;
(b) un antioxydant; et
(c) un mélange d'acides gras saturés et insaturés.

2. La composition selon la revendication 1, dans laquelle les cellules de mammifères comprennent des monocytes ou kératinocytes épidermiques.

3. La composition selon la revendication 1 ou 2, dans laquelle le pyruvate est sélectionné dans le groupe consistant en acide pyruvique, pyruvate de lithium, pyruvate de sodium, pyruvate de potassium, pyruvate de magnésium, pyruvate de calcium, pyruvate de zinc, pyruvate de manganèse et leurs mélanges.

4. La composition selon la revendication 3, dans laquelle le pyruvate est le pyruvate de sodium.

5. La composition selon les revendications 1 à 4, dans laquelle l'antioxydant est sélectionné dans le groupe consistant en rétinol, 3,4-didéhydrorétinol, alpha-carotène, béta-carotène, gamma-carotène, delta-carotène, acide ascorbique, alpha-tocophérol, béta-tocophérol, gamma-tocophérol, delta-tocophérol et les mélanges en dérivant.

6. La composition selon la revendication 5, dans laquelle l'antioxydant est l'alpha-tocophérol.

7. La composition selon les revendications 1 à 6, dans laquelle le mélange d'acides gras saturés et insaturés comprend l'acide laurique, acide myristique, acide myristoléique, acide pentadécanoïque, acide palmitique, acide palmitoléique, acide margarique, acide margaroléique, acide stéarique, acide oléique, acide linoléique, acide linolénique, acide arachidique et acide gaddoléique.

8. La composition selon la revendication 7, dans laquelle le mélange d'acides gras saturés et insaturés comprend les acides gras humains, de poulet, de veau, de mouton, de cheval, de porc et de baleine.

9. La composition selon les revendications 1 à 8, dans laquelle chacun des composants pyruvate, antioxydants et acides gras sont présents dans la composition thérapeutique en une quantité qui est d'environ 10 à environ 50 % par rapport au poids de la composition thérapeutique.

10. Un procédé de préparation d'une composition thérapeutique pour la prévention et l'atténuation des lésions des cellules de mammifères et l'augmentation de la vitesse en régénération des cellules de mammifères lésées qui comprend les étapes de mélange des ingrédients selon l'une quelconque des revendications précédentes.

11. Une composition pharmaceutique complète présentant une capacité améliorée à prévenir et atténuer les lésions des cellules de mammifères qui comprend :
(A) une composition thérapeutique selon l'une quelconque des revendications 1 à 9 ;
(B) un médicament utile pour le traitement des cellules de mammifères lésées.

12. La composition pharmaceutique complète selon la revendication 11, dans laquelle le médicament est sélectionné parmi les anti-inflammatoires, anti-bactériens, antiseptiques, les médicaments soulageant les brûlures, les médicaments pour le traitement des brûlures dues au soleil, les préparations traitant l'acné, les médicaments pour le traitement des piqûres et des morsures d'insectes, les agents lavant les blessures et les pansements pour blessures les médicaments pour les traumatismes, les médicaments utilisés pour les maladies auto-immunes, les médicaments traitant l'arthrite, les médicaments utilisés pour les ulcères et les médicaments utilisés pour le cancer.

13. Une composition pharmaceutique cytoprotectrice pour la prévention et l'atténuation des lésions des cellules de mammifères dues à un médicament présentant des propriétés cytotoxiques qui comprend :
(A) une composition thérapeutique selon l'une quelconque des revendications 1 à 9 ; et
(B) un médicament présentant des propriétés cytotoxiques.

14. Une composition pharmaceutique cytoprotectrice selon la revendication 13, dans laquelle le médicament présentant des propriétés cytotoxiques est sélectionné dans le groupe consistant en agents réduisant la cohésion des cellules épithéliales, abrasifs dermatologiques, anti-inflammatoires, agents de régulation lipidique, anti-cholinestérases à action centrale, médicaments chimiothérapeutiques et irritants gastriques.

15. La composition pharmaceutique cytoprotectrice selon la revendication 14, dans laquelle le médicament présentant des propriétés cytotoxiques est sélectionné dans le groupe consistant en gemfibrozil, lovastatine, tacrine et doxorubicine.

16. Une composition pharmaceutique pour la cicatrisation des blessures pour augmenter la vitesse en régénération des cellules de mammifères lésées qui comprend :
(A) un premier composant cicatrisant comprenant un mélange d'acides gras saturés et insaturés ; et
(B) un second composant cicatrisant consistant en :
(a) pyruvate sélectionné dans le groupe consistant en acide pyruvique, sel pharmaceutiquement acceptable de l'acide pyruvique et les mélanges en dérivant, et
(b) un antioxydant.

17. Un procédé de préparation d'une composition pharmaceutique complète présentant une capacité améliorée à prévenir et atténuer les lésions des cellules de mammifères qui comprend les étapes de :
(A) utilisation d'une composition thérapeutique sélectionnée dans le groupe consistant en :
(a) pyruvate sélectionné dans le groupe consistant en acide pyruvique, sel pharmaceutiquement acceptable de l'acide pyruvique et les mélanges en dérivant,
(b) un antioxydant ; et
(c) un mélange d'acides gras saturés et insaturés ;
(B) utilisation d'un médicament utile pour le traitement des cellules de mammifères lésées ; et
(C) mélange de la composition thérapeutique de l'étape (A) avec le médicament de l'étape (B) pour préparer la composition pharmaceutique complète.

18. Un procédé de préparation d'une composition pharmaceutique cytoprotectrice pour prévenir et atténuer les lésions des cellules de mammifères dues à un médicaments présentant des propriétés cytotoxiques qui comprend les étapes de :
(A) utilisation d'une composition thérapeutique sélectionnée dans le groupe consistant en :
(a) pyruvate sélectionné dans le groupe consistant en acide pyruvique, sel pharmaceutiquement acceptable de l'acide pyruvique et les mélanges en dérivant,
(b) un antioxydant ; et
(c) un mélange d'acides gras saturés et insaturés ;
(B) utilisation d'un médicament présentant des propriétés cytotoxiques ; et
(C) mélange de la composition thérapeutique de l'étape (A) avec le médicament de l'étape (B) pour préparer la composition pharmaceutique cytoprotectrice.

19. Un procédé de préparation d'une composition pharmaceutique cicatrisante pour augmenter la vitesse en régénération des cellules de mammifères lésées qui comprend les étapes de :
(A) utilisation d'une premier composant cicatrisant comprenant un mélange d'acides gras saturés et insaturés ; et
(B) utilisation d'un second composant cicatrisant consistant en :
(a) pyruvate sélectionné dans le groupe consistant en acide pyruvique, sel pharmaceutiquement acceptable de l'acide pyruvique et les mélanges en dérivant, et
(b) un antioxydant ; et
(C) mélange du premier composant cicatrisant de l'étape (A) avec le second composant cicatrisant de l'étape (B) pour préparer la composition pharmaceutique cicatrisante.

20. Un procédé de conservation des cellules de mammifères dans un milieu de culture qui comprend les étapes de :
(A) utilisation d'une composition thérapeutique sélectionnée dans le groupe consistant en :
(a) pyruvate sélectionné dans le groupe consistant en acide pyruvique, sel pharmaceutiquement acceptable de l'acide pyruvique et les mélanges en dérivant,
(b) un antioxydant ; et
(c) un mélange d'acides gras saturés et insaturés dans lequel les acides gras sont les acides gras requis pour la régénération des cellules de mammifères lésées ;
(B) introduction de cellules de mammifères dans un milieu de culture ; et
(C) mise en contact de la composition thérapeutique de l'étape (A) avec les cellules de mammifères présentes dans le milieu de culture provenant de l'étape (B).

21. Une composition pharmaceutique thérapeutique pour prévenir et atténuer les lésions des cellules de mammifères et augmenter la vitesse en régénération des cellules de mammifères lésées, qui comprend :
(A) une quantité thérapeutiquement efficace d'une composition thérapeutique selon l'une quelconque des revendications 1 à 9; et
(B) un support pharmaceutiquement acceptable.

22. La composition pharmaceutique selon la revendication 21, dans laquelle le support pharmaceutiquement acceptable est un système pharmaceutique.

23. La composition pharmaceutique selon la revendication 21, dans laquelle le support pharmaceutiquement acceptable est un véhicule topique.

24. La composition pharmaceutique selon la revendication 21, dans laquelle le support pharmaceutiquement acceptable est un véhicule pouvant être ingéré.

25. Un procédé de préparation d'une composition pharmaceutique thérapeutique pour prévenir et atténuer des lésions de cellules de mammifères et l'augmentation de la vitesse en régénération des cellules de mammifères lésées qui comprend les étapes de :
(A) utilisation d'une quantité thérapeutiquement efficace d'une composition thérapeutique selon l'une quelconque des revendications 1 à 9 ;
(B) utilisation d'un support pharmaceutiquement acceptable ; et
(C) mélange de la composition thérapeutique de l'étape (A) et du support pharmaceutiquement acceptable de l'étape (B) pour former une composition pharmaceutique thérapeutique.

26. Un procédé selon la revendication 25 pour la préparation d'une composition pharmaceutique thérapeutique pour prévenir et atténuer des lésions des cellules de mammifères et l'augmentation de la vitesse en régénération des cellules de mammifères lésées qui comprend les étapes de :
(A) utilisation d'une quantité thérapeutiquement efficace d'une composition thérapeutique sélectionnée dans le groupe consistant en :
(a) pyruvate sélectionné dans le groupe consistant en acide pyruvique, sel pharmaceutiquement acceptable de l'acide pyruvique et les mélanges en dérivant,
(b) un antioxydant ; et
(c) un mélange d'acides gras saturés et insaturés dans lequel les acides gras sont ceux requis pour la régénération des cellules de mammifères lésées;
(B) utilisation d'un support pharmaceutiquement acceptable ; et
(C) mélange de la composition thérapeutique de l'étape (A) et du support pharmaceutiquement acceptable de l'étape (B) pour former une composition pharmaceutique thérapeutique.
